(19)

Europäisches Patentamt
European Patent Office
Office européen des brevets

(11) **EP 4 372 100 A1**

(12) **EUROPEAN PATENT APPLICATION**

(43) Date of publication:
**22.05.2024 Bulletin 2024/21**

(21) Application number: **22306683.8**

(22) Date of filing: **15.11.2022**

(51) International Patent Classification (IPC):
***C12Q 1/6813*** [(2018.01)]    ***C12Q 1/6851*** [(2018.01)]

(52) Cooperative Patent Classification (CPC):
(C-Sets available)
**C12Q 1/6851; C12Q 1/6813**    (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Stilla Technologies**
**94800 Villejuif (FR)**

(72) Inventors:
• **FRADET, Etienne**
  **94110 ARCUEIL (FR)**
• **SANTOS BARRIOPEDRO, Irene**
  **91300 MASSY (FR)**
• **URSUEGUI, Sylvain**
  **69005 LYON (FR)**
• **DANGLA, Rémi**
  **75009 PARIS (FR)**

(74) Representative: **Regimbeau**
**20, rue de Chazelles**
**75847 Paris Cedex 17 (FR)**

Remarks:
The complete document including Reference Table(s) and the Sequence Listing(s) can be downloaded from the EPO website

(54) **DATA ANALYSIS METHOD FOR MULTIPLEX DIGITAL PCR USING COLOR-COMBINATION**

(57) The present application relates to multiplex digital polymerase chain reaction assays (dPCR assays), methods and systems in which Target Sequences are characterized by a combination of at least two colors (color-combination approach). The methods of the invention differ from the prior art methods in the analysis steps that are implemented to process the fluorescence recordings of said dPCR assays. In particular, they are characterized by the fact that only two categories of partitions are to be taken into account: 1) the total number of "all negative" partitions which have a low fluorescence level for all fluorophore types; and 2) the number of partitions displaying a high fluorescence level for all (but only) the fluorophore types corresponding to the color combination encoding the target sequence. This method is both simple to practice, and efficient in determining precisely the concentration of numerous target sequences (typically more than 10) in complex samples.

EP 4 372 100 A1

(52) Cooperative Patent Classification (CPC): (Cont.)

C-Sets
**C12Q 1/6813, C12Q 2531/113, C12Q 2537/143,
C12Q 2537/165, C12Q 2563/107,
C12Q 2563/159, C12Q 2565/102,
C12Q 2565/629;
C12Q 1/6851, C12Q 2537/143, C12Q 2537/165,
C12Q 2563/107, C12Q 2563/159,
C12Q 2565/102, C12Q 2565/629**

**Description**

TECHNICAL FIELD OF THE INVENTION

The present application relates to multiplex digital polymerase chain reaction assays (dPCR assays), methods and systems in which Target Sequences are characterized by a combination of at least two colors (color-combination approach). The methods of the invention differ from the prior art methods in the analysis steps that are implemented to process the fluorescence recordings of said dPCR assays. In particular, they are characterized by the fact that only two categories of partitions are to be taken into account: 1) the total number of "all negative" partitions which have a low fluorescence level for all fluorophore types; and 2) the number of partitions displaying a high fluorescence level for all (but only) the fluorophore types corresponding to the color combination encoding the target sequence. This method is both simple to practice, and efficient in determining precisely the concentration of numerous target sequences (typically more than 10) in complex samples.

BACKGROUND OF THE INVENTION

Digital polymerase chain reaction (dPCR) is a powerful and sensitive method that can be used to detect rare mutations in nucleic acid samples. This recently emerged technology yields quantitative information of unparalleled precision about nucleic acids. Its applications are diverse, ranging from clinical specialties such as oncology, for example for the genotyping and monitoring of lung cancer, an area with high research activity, organ transplantation, microbiology, virology or non-invasive prenatal testing; to environmental studies, or health and safety monitoring for food and feed products.

In many domains, whether in biology or in medicine, there is a need to extract as much information as possible from one given sample, ideally from a single experiment. This need for multiplex analysis can be driven by multiple reasons: rare availability of sample, or an effort to decrease costs of analysis. Hence, there is a need in digital PCR for detecting more than one or two targets at once.

Currently, most available commercial platforms only offer two-color detection, but have developed multiplexing strategies which involves manipulating probe and primer concentrations, in order to generate populations of different fluorescence amplitudes for a given detection channel [1,2].

A multiplexing strategy that ensures robustness of results is to use multiple distinct detection channels, to assign one target per detection channel and to take advantage of the many fluorophores available for nucleic acid detection by fluorescence. This is the " 1 color - 1 target" approach. For example, dPCR assays using allele-specific fluorescent TaqMan® probes require a dPCR instrument with R detection channels to detect R somatic mutations in biomarker genes. In such a dPCR duplex assay set up on a dPCR instrument with 2 detection channels, a first probe recognizing a first nucleic acid sequence (for example, a wild-type allele) and a second probe recognizing a second nucleic acid sequence (for example, a specific mutant allele) are used. Upon hybridization of a first probe or a second probe to an amplicon in a dPCR partition, the probe releases its fluorophore through the exonuclease activity of a DNA polymerase. The released fluorophore from the first probe is detected via a first fluorescence detection channel that is distinct from a second fluorescence detection channel that detects the released fluorophore from the second probe. Nonetheless, this "1 color - 1 target" approach is limited to detecting no more than R Target Sequences using a dPCR instrument with R detection channels [3].

One approach to increase the level of multiplexing has been described in the literature and is known as "intensity-based multiplexing" ([4]). This assay combines, for at least one of the fluorophore types used in the assays, two or more TaqMan® probe types that share the same fluorophore type but which have different target sequences. The two or more TaqMan® probe types that share the same fluorophore type are combined in the assay at two distinct concentrations. When using only 2 probe types per fluorophore type, one probe type would have a low concentration ("lo" type) and one probe type would have a high concentration ("hi" type). As a result, after PCR, if a partition contains the target for the "lo" type, the fluorescence level of the associated fluorophore type in the partition would be above a set positivity threshold but at a lower level than if the partition would contain the target for the "hi" type. After PCR, assuming that all targets are present in the sample, for each fluorophore type for which intensity-based multiplexing has been implemented, there would be negative population of droplets, a first "lo" positive population of droplets, a second "hi" positive population of droplets with a higher measured fluorescence level and possibly a third "lo+hi" positive population of droplets with an even higher measured fluorescence level (these would contain both the "lo" and "hi" targets such that the fluorescence signals add up). Using this approach, the different targets are distinguished by exploiting the measured fluorescence intensity of the partitions, in addition to an initial binary classification of the droplets according to set positivity thresholds.

This approach may not be entirely reliable in the presence of inhibitors, or of nucleic acids of poor quality that may affect differently the multiple targets leading to smears, displacements or fusions of partitions populations not observed for the controls onto which the assay was calibrated [5].

Another drawback of the intensity-based approach of Lindner et al. [4] is the complexity of the droplet classification and of the data analysis. As a matter of fact, with this approach, multiple thresholds must be set for each fluorophore type to distinguish between the multiple populations of positive partitions that all have distinct fluorescence intensities. For example, when using a 2-level intensity-based approach, three thresholds are required for each fluorophore type used to appropriately classify the partition populations and appropriately detect and quantify the targets of interest (one threshold for "lo" positive partitions, one threshold for the "hi" positive partitions and one threshold for the "lo+hi" positive partitions).

In view of this drawback, there remains a need for higher levels of multiplexing with less thresholds and a simpler analysis of the data.

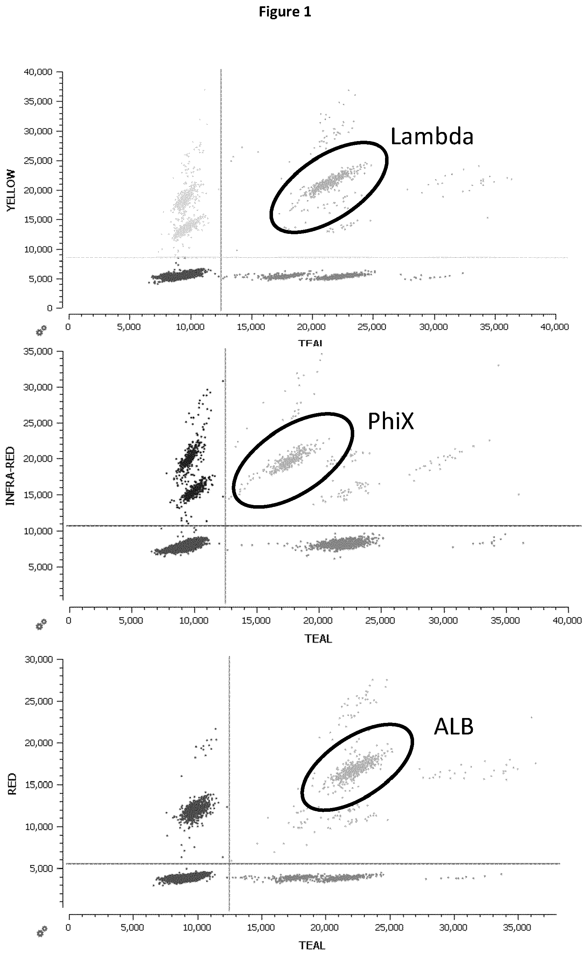

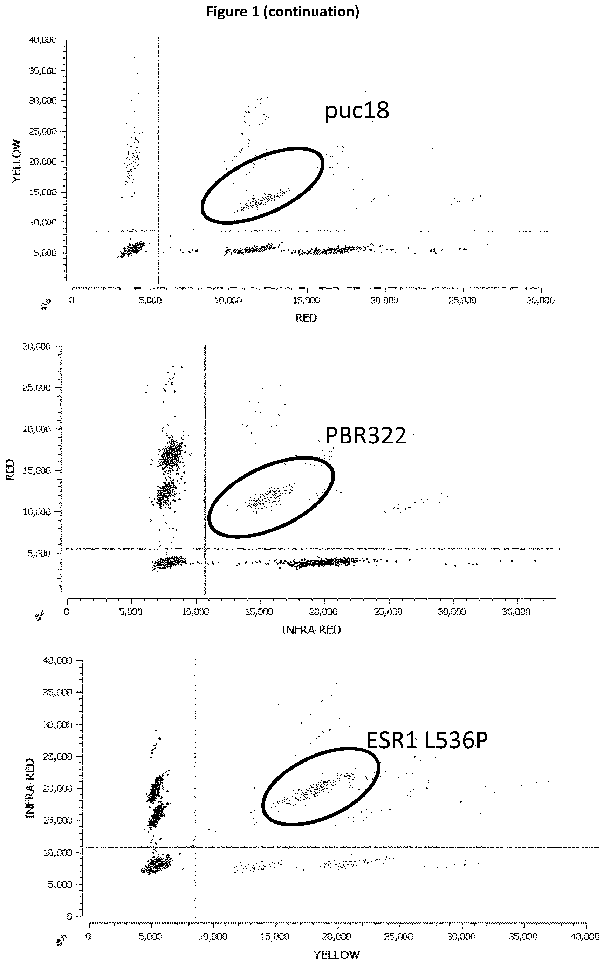

In this context, Marras et al. ([6]) ) proposed a real-time PCR method involving to color-code each target with two or more colors ("color-combination approach"). However, with this method, they only tested samples containing one Target Sequence (cf. figure 1 of Marras et al.). The authors acknowledged that the use of combinatorically labeled hybridization probes would generate results that are difficult to interpret, when multiple targets are present in the sample. This limitation is due to the fact that Marras et al. used real-time PCR, which means that, when multiple targets are present in a sample, they are amplified simultaneously in the same reaction, leading to competing parallel PCR reaction and modified fluorescent signals.

This limitation can be circumvented by using digital PCR (dPCR). In digital PCR, when multiple targets are present in a sample, they are separated in different partitions during the partitioning step, such that the likelihood of competing PCR reaction is greatly reduced. However, the implementation of color combination in digital PCR has not yet been demonstrated.

Nonetheless, co-encapsulation (i.e. partitions that contain at least 2 different Target Sequences) still exists in digital PCR and a subset of partitions will contain more than 1 type of Target Sequences. When using a "1 color - 1 target" approach, it is straightforward to deduct which Target Sequences are present in partitions which are positive for multiple fluorophore types since each color codes for a distinct type of Target Sequence. However, this task becomes highly challenging when at least one Target Sequence is color-coded using 2 or more fluorophore types. Partitions which are positive for more fluorophore types than the number of fluorophore types used for color-coding are ambiguous with regards to their content of Target Sequences. The treatment of such ambiguous partitions and the deconvolution of the measured fluorescence signals are complex. This complexity is moreover increased by the fact that, nowadays, commercial dPCR machines afford 5, 6 or more fluorescence detection channels. For example, a double color coding on a 6 channels dPCR instrument triggers the simultaneous monitoring and analysis of 15 different targets in the same sample.

In this context, data interpretation of the results generated by combinatorically labeled hybridization probes that are specific to multiple targets remains a challenge, especially when dPCR machines equipped with more than 3 fluorescent detection channels are used.

Thus, it remains a need to identify a color-combination approach that would allow to precisely and reproducibly quantify, by using dPCR machines containing more than 3 detection channels, the presence and concentration of a large number of Target Sequences (typically more than 10) in a complex sample.

The present invention solves this need, by providing a dPCR method which is both simple to practice, and efficient in determining precisely the presence and concentrations of numerous target sequences in samples, and that is applicable to a range of biologically meaningful sample sources.

DETAILED DESCRIPTION OF THE INVENTION

The present application provides a method for the detection and the quantification of a plurality of target sequences in nucleic acid samples using multiplex dPCR assays. This method can be used to detect different mutant target sequences. This method can also be used to assess microsatellite instability (MSI) and/or to detect genome-editing products.

The method of the invention contains the main step of conventional dPCR assays, namely:

a) contacting the sample with fluorophore-carrying probes capable of directly or indirectly binding to a Target Sequence (TS);

b) separating the sample into a set of partitions;

c) exponentially amplifying the TS in the presence of a PCR reaction mix;

d) measuring, for each partition, the intensity of fluorescence for each fluorophore type.

Definitions

As used herein, the term "digital PCR" refers to a PCR assay that separates a sample into a large number of partitions and where PCR reactions are carried out in each partition. Signal from each partition is detected to allow quantification of nucleic acids by statistical analysis. Currently available commercial digital PCR platforms rely on two distinct approaches. The first to be developed was chamber digital PCR, which relied on 2D arrays of microchambers to partition the sample [7]. Once filled with the PCR mix, the microchambers were thermocycled on a flat-block thermocycler, then imaged using fluorescence to reveal the amplified positive partitions. The second approach was droplet digital PCR, in which the sample was instead partitioned in a bulk emulsion of microdroplets using platform-specific consumables. The emulsion is transferred to a PCR tube or plate for thermocycling. After PCR amplification, data acquisition is performed in a process analogous to flow cytometry, whereby droplets are fluorescently read one by one as they pass in front of a single laser excitation source [8]. The naica® system performs digital PCR using a hybrid approach named "Crystal digital PCR™", combining the 2D array format and the use of droplet partitions. First, the samples are partitioned into 2D monolayer arrays of monodisperse droplets, called droplet crystals. These droplet crystals are then thermocycled on a flat-block thermocycler before being transferred onto a fluorescence microscope and imaged to reveal the amplified partitions. The whole process takes place inside a specifically designed microfluidic chip, such as the Sapphire chip or the Ruby chip, and requires the use of two instruments: i) the naica® Geode, which performs the sample partitioning and the thermal cycling of the droplet crystals, and ii) the naica® Prism, an automated fluorescence microscope equipped with 3 or 6 distinct fluorescence channels ([9]). Another approach for digital PCR performs fluorescence imaging at multiple steps during PCR amplification and the partitions are classified based on the obtained fluorescence curves. This is an hybrid between digital PCR and real-time PCR. All the known dPCR instruments can be used to implement the method of the invention, notably chamber digital PCR, droplet digital PCR, Crystal Digital PCR™, BEAMing (beads, emulsion, amplification, and magnetic)-based digital PCR, and microfluidic chip-based digital PCR.

As used herein, the term "partitioning" or "partitioned" refers to separating a sample into a plurality of portions, or "partitions". Typically, the sample is partitioned into at least 500 partitions. Partitions are generally physical, such that a sample in one partition does not, or does not substantially, mix with a sample in an adjacent partition. Partitions can be solid or fluid. In some embodiments, a partition is a solid partition, e.g., a microwell. In some embodiments, a partition is a fluid partition, e.g., a droplet. In some embodiments, a fluid partition (e.g., a droplet) is the result of a mixture of immiscible fluids (e.g., water and oil). In some embodiments, a fluid partition (e.g., a droplet) is an aqueous droplet that is surrounded by an immiscible carrier fluid (e.g., oil). As used herein, "substantially all partitions" refer to at least about any one of 70%, 75%, 80%, 85%, 90%, 95%, 98%, 99%, or more of the total number of partitions. The partitions described herein can be in any suitable format. Microwell plates, capillaries, oil emulsion, and arrays of miniaturized chambers with nucleic acid binding surfaces can be used to partition the samples. In a preferred embodiment, the partitions of the method of the invention are droplets. In a preferred embodiment, the naica® dPCR platform is used to carry out the partitioning step of the method of the invention. In some embodiments, the Sapphire chip of the naica® dPCR platform is used to partition the sample. Typically, a Sapphire chip contains 4 microchambers, each with a 2-dimensional monolayer of droplets. In some embodiments, the Ruby chip of the naica® dPCR platform is used to partition the sample. Typically, a Ruby chip contains 16 microchambers, each with a 2-dimensional monolayer of droplets. In some embodiments, data from dPCR reactions in droplets from different microchambers in a Sapphire chip is combined to provide quantification of the target sequences that the method is designed to detect.

In a preferred embodiment, the sample is partitioned into a sufficient number of partitions such that at least a majority of partitions has no more than 1-5 target regions or template molecules thereof (e.g., no more than about 1, 2, 3, 4, or 5 target regions or amplicons thereof). Specifically, the majority of partitions has more than 1 target regions or amplicons, but less than 3 target regions or amplicons in each.

Generally, partitions can contain an excess of enzyme, probes, and primers such that each mixture partition is likely to successfully amplify any target regions present in the partition.

In a preferred embodiment, the volume of all partitions is constant. The method of the invention can also be performed on partitions whose volume is not constant. In another embodiment, the distribution of the volume of the partitions around the mean volume is known prior to performing the method of the invention and the obtained results are corrected to account for this distribution around the mean. In another embodiment, the volume of the partitions is measured while performing the method, for example by using the fluorescence measurements obtained on the partitions, and the obtained results are corrected to account for the measure partition volumes.

Steps a) and b) of the method of the invention can be performed in any order. Thus, the sample can be first partitioned, and then the detection reagents (e.g., probes, enzyme, etc.) are afterwards incorporated into the partitioned samples. Alternatively, in a preferred embodiment, it is possible to first contact the samples with detection reagents (e.g., probes, enzyme, etc.) and then partition the sample. Preferably, in the method of the invention, the sample is partitioned shortly after mixing reagents together so that substantially all, or the majority, of reactions (e.g., DNA amplification, DNA cleavage, etc.) occur after partitioning. In other cases, the reagents are mixed in a state, for example at a temperature, in which reactions proceed slowly, or not at all. In this state, the sample is then partitioned, and lastly, the reaction is triggered to allow the reaction to proceed, for example by adjusting the temperature. Other triggers, such as optical or chemical triggers, could be used.

As used herein, the terms "polynucleotide" and "nucleic acid" are used interchangeably to refer to a polymer of nucleotides of any length. They include DNA and RNA. The nucleotides can be deoxyribonucleotides, ribonucleotides, modified nucleotides or bases, and/or their analogs, or any substrate that can be incorporated into a polymer by DNA or RNA polymerase. A polynucleotide may comprise modified nucleotides, such as Locked Nucleic Acids (LNAs), minor groove binders (MGBs), methylated nucleotides and their analogs, or any Tm-enhancer blockers or baits minimizing the selection of off-target nucleic acids. Nucleic acids may be single-stranded, double-stranded, or in more highly aggregated hybridization forms, and may include chemical modifications. The terms "polynucleotide" or "nucleic acid" may also be used to refer to the sequence encoded by the nucleic acid, including the sense strand (i.e., coding strand) sequence and anti-sense strand (i.e., noncoding strand) sequence in a double-stranded nucleic acid molecule.

As used herein, the term "target sequence" refers to a unique genomic location that defines the position of an individual nucleic acid sequence of interest, including one or more contiguous nucleotides. In some embodiments, the target sequence is a single nucleotide position of interest. In some embodiments, the target sequence is at least about any of 2, 3, 5, 10, 15, 20, 25 contiguous nucleotides. A gene may contain multiple target sequences of interest. The "target sequence" may refer to the sequence of either the sense strand or the anti-sense strand sequence of the target region. The target sequence can be any target nucleotide sequence of interest, for example a sequence displayed in the genome of a target pathogen, or a mutant sequence.

In the context of the invention, the "target sequence" is preferably a mutant sequence as defined below, said mutant sequence being present in low amount in the sample. In some embodiments, the target sequence is not the wild-type sequence.

As used herein, the terms "mutant sequence" and "variant sequence" as used interchangeably herein, refer to any sequence alteration in a sequence of interest in comparison to a reference sequence. It may be a purpose of the method of the invention to quantify their concentration in a sample. Mutant sequences include, but are not limited to, insertions, deletions, and substitutions, including single nucleotide changes, and alterations of more than one nucleotide in a sequence. Mutant sequences may be located at mutation hotspots, microsatellite sequence locii, or within site-specific genome-editing reagents. They may be SNVs or SNPs. "Mutation hotspots" refer to genetic loci that are known to be prone to naturally-occurring mutations, for example, in a diseased tissue or a diseased state. As used herein, the term "single nucleotide variant," or "SNV" for short, refers to the alteration of a single nucleotide at a specific position in a genomic sequence. When alternative alleles occur in a population at appreciable frequency (e.g., at least 1% in a population), a SNV is also known as "single nucleotide polymorphism" or "SNP". A "microsatellite sequence locus" herein refers to a region of genomic DNA that contains short, repetitive sequence elements of one to seven, such as one to five, or one to four base pairs in length. Each sequence repeated at least once within a microsatellite locus is referred to herein as a "repeat unit." Each microsatellite locus typically comprises at least seven repeat units, such as at least ten repeat units, or at least twenty repeat units. A "site-specific genome-editing reagent" refers to a component or set of components that can be used for site-specific genome editing. Generally, such a reagent contains a targeting module and a nuclease module.

The term "wildtype sequence" herein refers to a sequence to which one wishes to compare a sequence of interest, for example, a sequence corresponding to the dominant allele of a gene, or an unmodified sequence of a genetic locus. It is generally a sequence that is more abundant than mutant sequences in the sample to be tested.

As used herein, the terms "polymerase chain reaction" or "PCR" refers to a method in which a specific segment of a target double-stranded DNA is amplified. PCR is well known to those of skill in the art. Exemplary PCR reaction conditions typically comprise either two or three step cycles. Two-step cycles have a denaturation step followed by a hybridization/elongation step. Three step cycles comprise a denaturation step followed by a hybridization step followed by a separate elongation step. Also contemplated herein are polymerase chain reactions that are carried out without thermal cycling, including, but not limited to, rolling-circle amplification (RCA), isothermal PCR and loop-mediated isothermal amplification (LAMP).

A "primer" is generally a short single-stranded polynucleotide, generally with a free 3'-OH group, that binds to a target nucleic acid by hybridizing with a target sequence, and thereafter promotes polymerization of a polynucleotide complementary to the target nucleic acid. Primers can be of a variety of lengths and are often less than 50 nucleotides in length, for example 12-30 nucleotides, in length. Primers can be DNA, RNA, or a chimera of DNA and RNA portions. In some cases, primers can include one or more modified or non-natural nucleotide bases. In the context of the invention, each partition may comprise a plurality of primer sets corresponding to the plurality of target sequences. In some embodiments, each primer set comprises a forward primer and a reverse primer for amplifying the target sequence. In some embodiments, the forward primer and the reverse primer are oligonucleotide primers that anneal to opposite strands of a nucleic acid molecule and that flank the target sequence. The primer set allows production of an amplicon specific to the target fragment during the PCR reaction.

A nucleic acid sequence is "complementary" to another nucleic acid when at least two contiguous bases of, *e.g.,* a first nucleic acid or a primer, can combine in an antiparallel association or hybridize with at least a subsequence of a second nucleic acid to form a duplex. In some embodiment, complementary refers to hydrogen-bonded base pair formation preferences between the nucleotide bases G, A, T, C and U, such that when two given polynucleotides or nucleotide sequences anneal to each other, A pairs with T and G pairs with C in DNA, and G pairs with C and A pairs with U in RNA.

A first nucleic acid sequence "corresponding to" a second nucleic acid sequence is a sequence that is identical to or complementary to the second nucleic acid sequence or a portion of the second nucleic acid sequence, or comprises the second nucleic acid sequence or its complementary sequence. When a second nucleic acid sequence contains a unique feature, such as a mutation, a nucleic acid sequence "corresponding to" the second nucleic acid sequence comprises a sequence having the unique feature or a complement thereof.

As used herein, the terms "hybridization" and "annealing" refer to a reaction in which one or more polynucleotides react to form a complex that is stabilized via hydrogen bonding between the bases of the nucleotide residues. The hydrogen bonding may occur by Watson-Crick base pairing, Hoogstein binding, or by any other sequence specific manner. A nucleic acid, or a portion thereof, "hybridizes" to another nucleic acid under conditions such that non-specific hybridization is minimal at a defined temperature in a physiological buffer (*e.g.,* pH 6-9, 25-150 mM chloride salt). In some embodiments, the defined temperature at which specific hybridization occurs is room temperature. In some embodiments, the defined temperature at which specific hybridization occurs is higher than room temperature. In some embodiments, the defined temperature at which specific hybridization occurs is at least about 37, 40, 42, 45, 50, 55, 60, 65, 70, 75, or 80° C. In some embodiments, the defined temperature at which specific hybridization occurs is, or is about, 37, 40, 42, 45, 50, 55, 60, 65, 70, 75, or 80° C.

Each partition may comprise a polymerase, which is an enzyme that performs template-directed synthesis of polynucleotides, e.g., DNA and/or RNA. The term "polymerase" encompasses both the full-length polypeptide and a domain that has polymerase activity. DNA polymerases are well-known to those skilled in the art, including but not limited to DNA polymerases isolated or derived from *Pyrococcus furiosus, Thermococcus litoralis,* and *Thermotoga maritime,* or modified versions thereof. Additional examples of commercially available polymerase enzymes include, but are not limited to: Klenow fragment (New England Biolabs Inc.), Taq DNA polymerase (QIAGEN), 9° WM DNA polymerase (New England Biolabs Inc.), Deep Vent™ DNA polymerase (New England Biolabs Inc.), Manta DNA polymerase (Enzymat-ics), Bst DNA polymerase (New England Biolabs Inc.), and phi29 DNA polymerase (New England Biolabs Inc.). In some embodiments, the polymerase is a DNA-dependent polymerase. In some embodiments, the polymerase is an RNA-dependent polymerase, such as reverse transcriptase.

"Amplification" as used herein, generally refers to the process of producing two or more copies of a desired sequence. It can be performed by any polymerase chain reactions carried out with thermal cycling, or without thermal cycling, for example by rolling-circle amplification (RCA), isothermal PCR and loop-mediated isothermal amplification (LAMP) or by any known means. Components of an amplification reaction may include, but are not limited to, for example, primers, a polynucleotide template, polymerase, nucleotides, dNTPs and the like. An "amplicon" refers to a nucleic acid fragment formed as a product of a PCR amplification reaction that is a copy of a portion of a particular target nucleic acid, e.g., a target fragment comprising a target region. An amplicon will generally be double-stranded DNA, although reference can be made to individual strands thereof.

A droplet supports PCR amplification of template molecule(s) using homogenous assay chemistries and workflows similar to those widely used for real-time PCR applications ([10]). Once droplets are generated, they can be transferred on a PCR plate and emulsified PCR reactions can be run on a thermal cycler using a classical PCR program. Alternatively, droplets generated on a Sapphire chip or Ruby Chip of the naica® system can be subject to thermal cycling using a classical PCR program. Thermal cycling is performed to endpoint.

To circumvent the technical challenges associated with the amplification of low complexity sequence such as a microsatellite sequence, the annealing temperature and/or extension time of the amplification step may be increased. For example, typical annealing temperature is 55°C, and for microsatellite loci detection, the annealing temperature may be increased by an amount from 3 to 15°C.

As used herein, "specific", when used in the context of a primer specific for a target nucleic acid or a probe specific for a target nucleic acid, refers to a level of complementarity between the primer and the target such that there exists an annealing temperature at which the primer or probe will preferentially anneal to and mediate amplification and fluorescence detection of the target nucleic acid and will not anneal to or mediate amplification and fluorescence detection of non-target sequences present in a sample.

A "probe" herein refers to a molecule (*e.g.,* a protein, nucleic acid, aptamer, *etc.*) that specifically interacts with - or specifically binds to - a target polynucleotide. Non-limiting examples of molecules that specifically interact with or specifically bind to a target polynucleotide include nucleic acids (*e.g.,* oligonucleotides), proteins (*e.g.,* antibodies, transcription factors, zinc finger proteins, non-antibody protein scaffolds, *etc.*), and aptamers. Generally, a probe is labelled with a detectable label. The probe can indicate the presence or level of the target polynucleotide by either an increase or decrease in signal from the detectable label. In some embodiments, the probes detect the target polynucleotide in an amplification reaction by being digested by the 5' to 3' exonuclease activity of a DNA-dependent DNA polymerase. In the context of the invention, the probe is preferably a nucleic acid probe. In addition, the probe sequence can incorporate modified based such as Locked Nucleic Acid bases (LNA® bases), MGB, or other Tm-enhancers or means to minimize off-targeting. This probe can be a mediator probe (that does not carry any label) or a labelled probe. Examples thereof are provided below.

As used herein, "color-combination" means, in the context of digital PCR experiments, the concept of using more than one (1) fluorophore type for at least one target. The method of the invention requires that at least one Target Sequence (TS) is characterized by at least two different fluorophore types (or "color"). It is therefore hereafter called the "color combination approach" or "color combination method", as opposed to prior art dPCR methods in which each TS is associated to only one fluorophore type / color.

As used herein, the term "fluorophore type" corresponds to the chemical structure of a given fluorophore moiety. Several different fluorophore types can be used in the methods of the invention, such as FAM, VIC, Yakima Yellow@, HEX, ROX, Cy®5, ATTO®550, ATTO®700, etc... Similarly, several different quencher types can be used, such as TAMRA™, BHQ™1, BHQ™2, BHQ™3, etc...

The detection instrument or detection unit of a digital PCR system will use one or more fluorescence detection channels. A "fluorescence detection channel" usually combines an excitation channel which illuminates the sample with light filtered to contain only a narrow band of wavelengths (excitation wavelength) and an emission channel which collects the light emitted by the sample only within a narrow band of wavelengths distinct from the excitation wavelength (emission wavelength). Commercial digital PCR systems have detection instrument or detection units that acquire data (using point detectors, PMTs or 2D-image sensors) from one, or two, or three, or four, or five, or six or more different fluorescence detection channels. For simplification, the verb "to image" is used irrespectively of whether the collected data is from a point detector or an image sensor. In all cases, each fluorescence detection channel utilizes a distinct pair of excitation wavelengths and emission wavelengths. In the method of the invention, the detection instrument or detection units is preferably able to acquire data (using point detectors, PMTs or 2D-image sensors) from at least five, or six or more different fluorescence detection channels.

For example, the Prism6 instrument, which is the detection instrument of the Naica System from Stilla Technologies, has 6 different fluorescence detection channels. The "Blue", "Teal", "Green", "Yellow", "Red" and "Infrared" channels combine excitation channels of bandwidth 450-490nm, 509-519nm, 533-557nm, 564-586nm, 625-643nm, 645-695nm with emission channels of bandwidth 505-535nm, 530-551nm, 568-593nm, 600-640nm, 655-685nm, 708-753nm respectively.

When a fluorophore moiety is imaged using a detection unit with multiple detection channels, light emitted by the fluorophore moiety will be principally detected in one of the detection channels. For example, a FAM fluorophore moiety will be principally detected in the "Blue" detection channel of a Prism6 instrument. However, light emitted by the fluorophore moiety may also be detected in other detection channels, a phenomenon called "fluorescence spill-over". For example, a FAM fluorophore moiety will also be detected in the "Teal" detection channel of a Prism6 instrument, although at lower signal intensities than in the "Blue" detection channel. The distribution of the light detected from a given fluorophore type across all fluorescence detection channels is called the fluorescence signature of the said fluorophore type.

In the method of the invention, the PCR data collection step is preferably performed using an optical detector having a high number of detection channels, e.g., a six-color detection system (for example, the naica® Prism6 system by Stilla, or the Qiagen Qiacuity 5-colors system). On a given fluorescence detection unit (like a Prism6), different fluorophore types have different fluorescence signatures.

The term "sample" as used herein refers to a sample that can be subject to the methods described herein with or without pre-processing such as nucleic acid extraction, fragmentation, dilution/concentration, or other pre-treatment. The sample may be a biological sample, or obtained by processing or manipulating a biological sample, such as a biological fluid or a biological tissue. Said biological sample preferably contains for example a tumor tissue, disseminated cells, feces, blood cells, blood plasma, serum, lymph nodes, urine, saliva, semen, stool, sputum, cerebrospinal fluid, tears, mucus, pancreatic juice, gastric juice, amniotic fluid, cerebrospinal fluid, or serous fluid. Alternatively, it can be an environmental sample such as sewage water.

In some embodiments, the sample as collected is ready for loading onto a digital PCR instrument for analysis. This is usually the case when the Target Sequences is in a very low amount in the sample, for example when it is a mutated sequence in a tumour sample.

In a preferred embodiment, however, the concentration of the nucleic acid molecules present in the sample has to be adjusted, e.g., by dilution of the sample or by concentrating the sample (e.g., by dialysis, or by lyophilization and reconstitution), to provide a suitable concentration for dPCR. In particular, it is important to dilute the sample for diminishing the quantity of sequences that are present in high quantity in the sample (e.g., WT sequences). In some embodiments, the method is carried out with a first sample, and the concentration of the nucleic acid molecules in the sample is adjusted based on a count of partitions that each produces a positive signal via three or more detection channels, wherein if (e.g., when) the count is larger than a pre-determined value, the adjusting is decreasing the concentration of the nucleic acid molecules in the sample by diluting the sample; or wherein if (e.g., when) the count is smaller than a pre-determined value, the adjusting is increasing the concentration of the nucleic acid molecules in the sample by concentrating the sample. In some embodiments, the dilution factor or the concentration factor is based on the count of partitions that each produces a positive signal via three or more detection channels. In some embodiments, the concentration of the nucleic acid molecules in the sample is adjusted based on the estimated concentration of the wildtype sequence, the estimated concentration of the mutant sequences, or the estimated concentration of the specific allelic sequences at one or more of the plurality of target regions in the sample. In some embodiments, the method is repeated with the sample diluted to one or more concentrations in order to provide optimal concentrations for accurate quantification of different genetic species (e.g., wildtype, mutant and/or allelic sequences) at different target regions.

In one preferred embodiment, the concentration of the sample is adjusted so that the maximum concentration of the Target Sequence before partitioning the sample is at least about any one of 50, 75, 100, 150, 200, 250, 500, 1000, 2000, 5000 or more copies per $\mu$L. It is preferably of at least about 150 to 500 copies per $\mu$L.

The method of the invention preferably contains a step of calculating a "fluorescence compensation matrix" or a "spill-over compensation matrix" using reference samples. This step is to be performed prior to running samples in digital PCR experiments that combine different fluorophore types. The importance of this step is well known in the art. It is due to the fact that fluorescence detection units do not directly measure the fluorescence level from individual fluorophore types. Indeed, a fluorescence detection unit measures the light level received by each fluorescence detection channel from each partition. The light level in one fluorescence detection channel is the sum of the fluorescence signals from all fluorophore types, each fluorophore having a distinct contribution defined by its fluorescence signature. Usually, one fluorophore type is the main contributor for the fluorescence signal detected in a given fluorescence detection channel. Nonetheless, other fluorophore types can contribute to the total signal. For example, when using FAM, Yakima Yellow@ and ATTO®550 fluorophore types conjointly on a Prism6 instrument, Yakima Yellow® is the main contributor to the signal detected in the "Teal" channel, but FAM and ATT®550 fluorophores also have secondary signals that add up to the Yakima Yellow@ signal in the "Teal" channel. Overall, the fluorescence signal in one fluorescence detection channel is thus the sum of, for each fluorophore type combined in the experiment, the fluorophore signal intensity times the fluorescence signature of said fluorophore in said channel. Plus an additional signal from the background, the "background fluorescence".

When using $n$ distinct fluorescence detection channels and $M$ different fluorophore types in one experiment, for each partition, $n$ signals are acquired that are each the sum of $M$ contributions from $M$ fluorophore types (plus "background fluorescence"). In practice, the useful information is the signal intensity $I(m)$ from each fluorophore type. Mathematically, this translates to $n$ linear equations with $M$ unknown variables. This mathematical problem can only be solved if:

    i) there are no more than $n$ fluorophore types combined in the experiment (M $\leq$ n);
    ii) all fluorophore types have distinct fluorescence signatures one from another.

As a result, only fluorophore types with distinct fluorescence signatures on a given fluorescence detection unit should be combined in one digital PCR experiment or digital PCR assay. In that case, a "fluorescence compensation matrix" or "spill-over compensation matrix" can be applied to transform the $n$ signals from the $n$ fluorescence detection channels into the $M$ fluorescence levels from the $M$ different fluorophore types. The "fluorescence compensation matrix" is a $n \times (n + 1)$ matrix, the "+1" being related to the supposedly known "background fluorescence".

Prior to running samples in digital PCR experiments that combine different fluorophore types, the fluorescence compensation matrix is preferably calculated using reference samples. Usually, "no template control (NTC)" samples and "monocolor control" samples are used to estimate the fluorescence compensation matrix. The NTC samples allow to estimate the "background fluorescence" and the "monocolor control" samples, each containing only one fluorophore type, are used to estimate the fluorescence signature of each fluorophore type across all fluorescence detection channels. Such control experiments allow for the estimation of the fluorescence compensation matrix but the estimation may be imperfect such that the conversion of the $n$ signals from the $n$ fluorescence detection channels to the $M$ signals from the $M$ fluorophore types will also be imperfect, leading to uncertainty and error in the measurement of the fluorophore signal intensities. More complex algorithms can be used to analyze the data taking into account such possible signal uncertainties.

In the following sections, unless specified differently, it is recommended that:

-    there are no more than $n$ fluorophore types combined in the experiment (M $\leq$ n);
-    all fluorophore types have distinct fluorescence signatures one from another;

- a spillover compensation has been estimated from control samples.

It would also be possible to use more than $n$ fluorophore types in some embodiments but only if the fluorophore types can be grouped into $n$ sets of fluorophore types, where within each set the different fluorophore types have similar fluorescence characteristics and can be treated indifferently while practicing the method of the invention. For example, FAM / AlexaFluor488 / Atto495 could be treated as one such set of fluorophore types.

As a result, for each partition, the fluorescence signal intensity from the $M$ fluorophore types is preferably measured using the $n$ signals from the $n$ fluorescence detection channels, by applying the fluorescence compensation matrix that has been generated beforehand.

Reference to "about" a value or parameter herein includes (and describes) variations that are directed to that value or parameter per se. For example, description referring to "about X" includes description of "X". For example, a value of about X may be within (i.e., $\pm$) 10%, 5%, 2%, 1% or less of X.

Description of the method of the invention

General principle of the color-combination analysis of the invention

In a first aspect, the present invention pertains to a multiplexed digital PCR (dPCR) method to detect and/or quantify, in a biological sample comprising nucleic acid molecules, the presence of a set of at least five different nucleic acid target sequences (TS), said method comprising:

a) contacting the sample with fluorophore-carrying probes capable of directly or indirectly binding to the TS; wherein each Target Sequence $i$ (TS$i$) is characterized by $k_i$ different fluorophore types, $k_i$ being superior or equal to 2;

b) separating the sample into a set of partitions;

c) exponentially amplifying the TS in the presence of a PCR reaction mix;

d) measuring, for each partition, the intensity of fluorescence for each fluorophore type;

e) counting, for each fluorophore type,

- the partitions having a fluorescence signal below a positivity threshold for the said fluorophore type,

- the partitions having a fluorescence signal above said positivity threshold for the said fluorophore type,

So as to determine:

- $N_0$ = the total number of partitions having a fluorescence signal below said positivity threshold for all the fluorophore types,
- $N_i$ = the total number of partitions having a fluorescence signal above the positivity threshold only for the $k_i$ fluorophore types characterizing TS$i$.

f) processing the data collected in step e) in order to quantify the concentration of the at least five TS$i$ in said biological sample.

In some embodiments, the positivity threshold for each fluorophore type is pre-defined. In some embodiment, the positivity thresholds are set while implementing the method. In some embodiments, the positivity thresholds are pre-defined and adjusted while implementing the method.

In a preferred embodiment, the positivity threshold of each fluorophore type is defined and/or adjusted such that it is above the cluster of partitions with the lowest fluorescence intensity, corresponding to the "all negative cluster" and below any cluster of partitions with a higher level of fluorescence intensity than the "all negative cluster".

In the method of the present invention, the number of fluorophore types used for characterizing each Target Sequence (TS) is designated by the letter "k". More precisely, in the method of the invention, each Target Sequence $i$ (TS$i$) is characterized by $k_i$ different fluorophore types, $k_i$ being superior or equal to 2.

In a preferred embodiment, $k_i$ is the same for each and all the TS to be detected in the experiment. For example, each and all the TS of the experiment are characterized by two (and only two) fluorophore types. Or each and all the TS of the experiment are characterized by three (and only three) fluorophore types.

In this embodiment, only the partitions having a fluorescence signal above the positivity threshold for at most

$k_i$ (and not more than $k_i$) fluorophore types should be taken into account in the method of the invention. Consequently, the partitions having a fluorescence signal above the positivity threshold for $k_i$+1 (or $k_i$+2 etc.) fluorophore types should not be taken into account in the method of the invention. For example, if each Target Sequence TSi is characterized by $k_i$=3 fluorophore types, then the partitions having a fluorescence signal above the positivity threshold for 4, 5, etc. fluorophore types should not be taken into account in the method of the invention.

More precisely, in the method of the invention, only the partitions having a fluorescence signal above the positivity threshold for the $k_i$ fluorophore types characterizing uniquely the TSi should be taken into account as "positive partitions". All the other partitions should be considered as "negative partitions" or should be excluded from the analysis. For example, if a TS is characterized by three fluorophore types FAM, Yakima Yellow® and ATTO®550, only the partitions having a fluorescence signal above the positivity threshold for only FAM, Yakima Yellow® and ATTO®550 will be held as "positive" for this TS. The partitions which are negative for all fluorophore types will be held as "negative". All the partitions positive for only a subset of the FAM, Yakima Yellow® and ATTO®550 fluorophores (e.g., FAM only; FAM and Yakima Yellow® only; Yakima Yellow® and ATTO®550only; FAM, Yakima Yellow®, Cy®5 and ATTO®550,etc.) can be held as "negative" for this TS. They can also not be taken into account in the analysis - they are ignored. All the other partitions, which would be positive for any fluorophore types other than FAM, Yakima Yellow® and ATTO®550 will not be taken into account in the analysis - they are ignored for this TS.

In another embodiment, $k_i$ is different for each TS to be detected in the experiment. For example, some TS are characterized by two (and only two) fluorophore types, whereas other TS are characterized by three (or four) fluorophore types.

Also in this case, for a Target Sequence TSi characterized by $k_i$ fluorophore types, the partitions having a fluorescence signal above the positivity threshold for $k_i$+1 fluorophore types should not be taken into account in the method of the invention. For example, if a Target Sequence TSi is characterized by $k_i$=3 fluorophore types, then the partitions having a fluorescence signal above the positivity threshold for 4 fluorophore types should not be taken into account in the method of the invention for this TSi. And, if a Target Sequence TSj is characterized by $k_j$=2 fluorophore types, then the partitions having a fluorescence signal above the positivity threshold for 3 fluorophore types should not be taken into account in the method of the invention for this TSj.

In the context of the invention, and as proposed above, the total number of different channels or fluorophore types or wavelength or signals that can be unambiguously detected by the dPCR instrument used in the proposed method is designated by the letter "n".

Preferably, the number $n$ of different fluorescence detection channels available for analysis in the method of the invention is superior or equal to 4, preferably superior or equal to 5, more preferably is 6 or 7. It can also be superior to 7.

In the context of the invention, the total number of different fluorophore types that are carried by all the probes used in the assay (i.e. the total number of the different fluorophore types that are combined in the experiment) is designated by the letter "M".

In a preferred embodiment, as explained above, $M$ is inferior or equal to $n$.

The method of the invention mainly distinguishes from prior art methods in that it relies, on step e), on a binary "negative or positive" classification of partitions for each fluorophore type, based on the measured fluorescence level of the partition for the said fluorophore type. Therefore, in the method of the invention, the precise fluorescence intensity value of the partition does not matter, as long as it is above the positivity threshold or below. In the present invention, this threshold is only used to differentiate positive partitions from negative ones.

As used herein, the "positivity threshold" corresponds to the intensity value distinguishing partitions displaying a high intensity of one fluorophore type from those displaying a low intensity of the same fluorophore type.

In a preferred embodiment, it is defined on 1D plot views, for each fluorophore type separately. It can also be defined on 2D plot views, whatever is the more appropriate for the skilled person.

Once this positivity threshold is set for each fluorophore type, it is easy to determine:

- the number "No" corresponding to the total number of partitions having a fluorescence signal below said positivity threshold for all the fluorophore types,

- the number "N$i$" corresponding to the total number of partitions having a fluorescence signal above the positivity threshold only for the $k_i$ fluorophore types characterizing TSi.

Based on these numbers, it is possible to quantify the concentration $C_i$ of the at least five TSi in said biological sample, according to classical Poisson's law.

For example, it is possible to quantify the concentration of the at least five TSi in said biological sample by using the following equation:

$$C_i = -\frac{d}{v}\ln(1 - \frac{N_i}{N_0 + N_i})$$

where v is the volume of the partition (e.g., a droplet) assumed to be constant, and d is the dilution factor used to dilute the biological sample to the microfluidic well (or any other partition recipient) where dPCR is performed.

In a particular embodiment, step e) of the method of the invention further contains the determination of the number "$N_1$" corresponding to the total number of partitions having a fluorescence signal above the positivity threshold for only one fluorophore type. This number $N_1$ can be determined in two different cases:

1) when an abundant sequence (e.g., WT) is detected in a single color (see the detail of this embodiment below, and example 4).

In this first case, $N_1$ is to be taken into account as "$N_i$" in the calculation of the concentration of said abundant sequence in the sample.

2) when no sequence is detected in a single color in the assay.

In this second case, $N_1$ can be taken into account in the calculation of the concentration of TSi in the sample, and treated in the analysis as being "negative partitions" (as the number "No"). In this latter case, for each target sequence i, the concentration $C_i$ can then be calculated using the following equation:

$$C_i = -\frac{d}{v}\ln(1 - \frac{N_i}{N_0 + N_1 + N_i})$$

where v is the volume of the partition (e.g., a droplet) assumed to be constant, and d is the dilution factor used to dilute the sample from the biological sample to the microfluidic well (or any other partition recipient) where dPCR is performed.

In another embodiment, $N_1$ is excluded from the analysis and treated as "artifact partitions" or "noise partitions". In this case, for each target sequence i, the concentration $C_i$ can then be calculated using the following equation:

$$C_i = -\frac{d}{v}\ln(1 - \frac{N_i}{N_0 + N_i})$$

More generally, step e) of the method of the invention can contain the determination of the number "$N_j$" with j being an integer comprised between 1 and $k_i$ ($1 < j < ki$) corresponding to the total number of partitions having a fluorescence signal above the positivity threshold for j fluorophore types. If no sequence is detected in j-colors in the assay, Nj can be taken into account in the calculation of the concentration of TSi in the sample, and treated in the analysis as being "negative partitions" (as the number "No"). In this latter case, for each target sequence i, the concentration Ci can then be calculated using the following equation:

$$C_i = -\frac{d}{v}\ln(1 - \frac{N_i}{N_0 + \sum N_j + N_i})$$

where v is the volume of the partition (e.g., a droplet) assumed to be constant, and d is the dilution factor used to dilute the sample from the biological sample to the microfluidic well (or any other partition recipient) where dPCR is performed.

Alternatively, Nj can be excluded from the analysis and treated as "artifact partitions" or "noise partitions". In this case, for each target sequence i, the concentration Ci can then be calculated using the following equation:

$$C_i = -\frac{d}{v}\ln(1 - \frac{N_i}{N_0 + N_i})$$

For a dPCR experiment using color-encoding with *n* distinct fluorescence detection channels and thereby a maximum of $M \leq n$ distinct fluorophore types, the number of different targets possible to measure independently using the color combination approach according to the invention will be given by the following equation, where *k* indicates the number of fluorophore types used to encode each one of the targets:

$$C_M^k = \frac{M!}{k!\,(M-k)!} \leq \frac{n!}{k!\,(n-k)!}$$

As an example, using a dPCR instrument equipped with 6 different detection channels, when the color-combination method of the invention uses k=2 different fluorophore types for each target, then the total number of distinct targets that can be detected and quantified will be 15 $(C_6^2 = 15)$. And if the invention uses k=3 different fluorophore types for each target, then the total number of distinct targets that can be detected and quantified will be $20\ (C_6^3 = 20)$.

If the dPCR instrument is equipped with 7 different detection channels, when the color-combination method of the invention uses k=2 different fluorophore types for each target, then the total number of distinct targets that can be detected and quantified will be $21\ (C_7^2 =$ 21). And if the invention uses k=3 different fluorophore types for each target, then the total number of distinct targets that can be detected and quantified will be $35\ (C_7^3 = 35)$.

Thus, the method of the invention enables to detect and/or quantify the presence of at least 10, preferably at least 15, more preferably at least 20, even more preferably at least 30 different TS in a biological sample.

In a particular embodiment, the method of the invention also enables to detect and/or quantify another set of Target Sequences (herein called $TS_e$), this time with a "one color-one sequence approach", i.e., each $TS_e$ being characterized (encoded) by only one fluorophore type.

Two cases are then to be distinguished: either this only one fluorophore type is also used for characterizing the set of TSi (i.e., it is used in the color-combination approach according to the invention); or this only one fluorophore type is not utilized to characterize the set of TS*i*.

In the first case, the step a) of the method of the invention uses $k_i$ different fluorophore types to detect a first set of at least five Target Sequences TS*i*, and one and only one of these $k_i$ fluorophore types is also used to detect one other Target Sequence $TS_e$ said $TS_e$ being characterized by only one fluorophore type. In other words, in this embodiment, one flurorophore type is used in combination with several other fluorophore types to detect one or more TS*i*, and as a single color to detect a Sequence $TS_e$.

In this case, the method of the invention enables to detect and/or quantify, in a biological sample comprising nucleic acid molecules, the presence of a first set of at least five different nucleic acid Target Sequences (TS*i*), and one nucleic acid Target Sequence ($TS_e$), and contains the following steps:

a) contacting the sample with fluorophore-carrying probes capable of directly or indirectly binding to the TS; wherein each of the at least five Target Sequence *i* (TS*i*) is characterized by $k_i$ different fluorophore types, $k_i$ being superior or equal to 2, and wherein the one sequence $TS_e$ is characterized by only one fluorophore type, this latter fluorophore type being one of the fluorophore types used to detect at least one TS*i*;

b) separating the sample into a set of partitions;

c) exponentially amplifying the TS in the presence of a PCR reaction mix;

d) measuring, for each partition, the intensity of fluorescence for each fluorophore type;

e) counting, for each fluorophore type,

- the partitions having a fluorescence signal below a positivity threshold for the said fluorophore type,

- the partitions having a fluorescence signal above said positivity threshold for the said fluorophore type,

So as to determine:

- $N_0$ = the total number of partitions having a fluorescence signal below said positivity threshold for all the fluorophore types,
- $N_i$ = the total number of partitions having a fluorescence signal above the positivity threshold only for the $k_i$ fluorophore types characterizing TS*i*.

- $N_e$ = the total number of partitions having a fluorescence signal above the positivity threshold only for the fluorophore type used to characterized $TS_e$,

f) processing the data collected in step e) in order to quantify the concentration of the at least five TSi and $TS_e$ in said biological sample.

In this embodiment, only the partitions having a fluorescence signal above the positivity threshold for at most $k_i$ (and not more than $k_i$) fluorophore types should be taken into account in the method of the invention. Consequently, the partitions having a fluorescence signal above the positivity threshold for $k_i+1$ (or $k_i+2$ etc.) fluorophore types should not be taken into account in the method of the invention. For example, if each Target Sequence TSi is characterized by $k_i$=3 fluorophore types, then the partitions having a fluorescence signal above the positivity threshold for 4, 5, etc. fluorophore types should not be taken into account in the method of the invention.

In this embodiment, it is possible to quantify the concentration in said biological sample of

- $TS_e$ by using the following equation:

$$C_e = -\frac{d}{v}\ln(1 - \frac{N_e}{N_0 + N_e})$$

- the TSi which are characterized using the fluorophore used to characterize $TS_e$ (and k-1 other fluorophores) by using the following equation:

$$C_i = -\frac{d}{v}\ln(1 - \frac{N_i}{N_0 + N_e + N_i})$$

- the TSi which are characterized using fluorophores other than the one used to characterize $TS_e$ by using the following equation:

$$C_i = -\frac{d}{v}\ln(1 - \frac{N_i}{N_0 + N_i})$$

where v is the volume of the partition (e.g., a droplet) assumed to be constant, and d is the dilution factor used to dilute the biological sample to the microfluidic well (or any other partition recipient) where dPCR is performed.

In the second case, the method of the invention enables to detect and/or quantify, in a biological sample comprising nucleic acid molecules, the presence of two different sets of TS:

- one set of at least five TSi according to the color-combination method of the invention, as defined above and below,

- one set of TS containing one or more Target Sequences $TS_e$ which are characterized by fluorophore types, said fluorophore types being not used to characterize the set of TS*i*.

In this case, the step a) of the method of the invention is defined as :

a) contacting the sample with fluorophore-carrying probes capable of directly or indirectly binding to two sets of TS; wherein for a first set of Target Sequences, each Target Sequence *i* (TS*i*) is characterized by $k_i$ different fluorophore types, $k_i$ being superior or equal to 2, and wherein a second set of Target Sequences is characterized by other fluorophore types not utilized to characterize the first subset of TS*i;*

The other steps of the method of the invention are not changed.

In particular, the calculations of Ci takes into account the numbers $N_i$ and $N_o$ as defined above, and the formula:

$$C_i = -\frac{d}{v}\ln(1 - \frac{N_i}{N_0 + N_i})$$

where v is the volume of the partition (e.g., a droplet) assumed to be constant, and d is the dilution factor used to dilute the biological sample to the microfluidic well (or any other partition recipient) where dPCR is performed.

One of the first steps of the method of the invention consists in contacting the sample with fluorophore-carrying probes that are capable of directly or indirectly binding to the TS.

The skilled person can use any probes that can be coupled to a fluorophore and bind directly or through another molecule (usually not labelled, such as a mediator probe) to a TS.

Examples of fluorophore-carrying probes that are capable of directly binding to a TS are TaqMan® probes.

Examples of fluorophore-carrying probes that are capable of indirectly binding to a TS are molecular beacons or Complementary Single Stranded Fluorescent Reporters (CSSFR) described herein. These reporter molecules are usually associated to mediator probes that can directly bind to the TS. The mediator probes can also bind to the reporter molecules.

Two preferred systems that can be used in the method of the invention are hereafter described.

TaqMan® probes

As used herein, a "TaqMan® probe" consists in an oligonucleotide sequence, called the "probe sequence" or the "target sequence", on which a fluorophore moiety and a quencher are covalently attached. Usually, the fluorophore moiety is attached to the 5'-end of the probe and the quencher is covalently attached at the 3'-end of the probe.

Each TaqMan® probe is composed of a sequence which is complementary to a target sequence, a type of fluorophore and a type of quencher. All TaqMan® probe types combined in the experiment will differ at least in either the target sequence or the type of fluorophore used.

In one embodiment, data for the method of the invention is generated using at least two, four, six, eight, ten or twenty TaqMan® probes, each carrying a different fluorophore type, the combination of which will characterize the target sequences in a unique manner.

The color combination method of the invention requires the use of two or more different TaqMan® probes for each target sequence. For each target sequence, the two or more different TaqMan® probes used must have different fluorophore types and may have different nucleotide sequences or quenchers.

In the case where, for a given target, the two or more different TaqMan® probes used have different and non-overlapping nucleotide sequences, the TaqMan® probes are said to be "non-competing". In the case where the two or more TaqMan® probes share the same nucleotide sequence, the TaqMan® probes are said to be "competing".

In a particularly preferred embodiment, the method of the invention uses at least two competing TaqMan® probes, i.e., at least two TaqMan® probes carrying different fluorophore types and containing the same nucleotide sequence that is complementary to the same Target Sequence.

When using "competing" TaqMan® probes, the concentrations of the two probes in the reaction mix may need to be adjusted in order for both probes to be cleaved at similar rates during PCR. Indeed, if the two probes are at equal concentrations and if one competing TaqMan® probe binds more preferentially to the target sequence during PCR, this said TaqMan® probe will be cleaved preferentially at each cycle of the PCR reaction. This can lead to an increase in the fluorescence level of only the "more preferential" TaqMan® probe, masking the signal from the other probe. By lowering the concentration of the "more preferential" probe relative to the "other probe", the effective binding efficiency of the two probes at each PCR cycle is equilibrated. As a result, fluorescence level from both fluorescence probe types can then be detected.

In one embodiment of the invention, the use of competing TaqMan® probes to effect color combination involves, in a first step, the design of an assay with one single Target Sequence instead of a multiplicity of Target Sequences. This first step is used to check whether, for both colors encoding the single Target Sequence, the fluorescence signal of positive partitions is sufficiently well separated from the fluorescence signal of negative partitions. This verification is performed using fluorescent probe types separately and also using a mixture of both fluorescent probe types. If, when using the mixture of both fluorescent probe types, at least one of the two fluorescence signals from the two colors encoding the single Target Sequence is not sufficiently well separated, the probe concentrations of the mixture of colors can be adjusted such that the fluorescence signals from both fluorescent probe types used in the encoding of the single Target Sequence are sufficiently well separated.

Example 1 below discloses how to use such TaqMan® probes in the method of the invention.

Mediator probes / fluorescent reporters

In other embodiments, instead of TaqMan® probes, the data for the method of the invention are generated by the use of fluorophore-carrying probes that indirectly detect Target Sequences. In this embodiment, said fluorophore-carrying probes are called "fluorescent detection molecules". These molecules do not bind to the Target Sequences by themselves. They however contain a tag complementary sequence which is complementary to a sequence present on a mediator probe, said mediator probe being able to directly bind to the TS. Such embodiments therefore involve mediator probes that are able to bind directly to the Target Sequences.

In a particular embodiment of competing mediator probes, a set of at least two mediator probes types and at least two fluorescent detection molecules are designed for each target sequence TSi. In the "color combination" method of the invention, each fluorescent detection molecule is associated to a distinct fluorophore type so that each target sequence is eventually associated to at least two fluorophore types.

More precisely, for each target sequence TSi, the set contains at least two mediator probes, each mediator probe containing:

◦ a 3' terminal probe region which is complementary to the target sequence TSi;
◦ a 5' terminal mediator region containing at least one tag sequence (tag) ;
◦ a biological cleavage site located between the mediator region and the probe region, whereby an enzyme with exonuclease activity can mediate the cleavage of the two regions during the amplification process of the target sequence.

wherein, in each set, the 3' terminal probe regions of the at least two mediator probes display the same sequences that are complementary to the TSi, and the 5' terminal mediator regions of the at least two mediator probes contain different tag sequences, that are able, once cleaved, to activate a different signal (e.g., to bind to different fluorophore-carrying probes).

And, for each target sequence TSi, the set contains at least two fluorescent detection molecules, said molecules containing at least :

◦ a single sequence (tag complementary sequence - TCS) that is complementary to and hybridize with at least one of the tag sequence (tag) located in the 5' terminal mediator region of one of the mediator probe of the set, and
◦ a quencher group and a fluorophore whose fluorescence is modified as a result of the hybridization or of the elongation of the tag sequence of the 5' terminal mediator region, once cleaved, on the tag complementary sequence (TCS) on the detection molecule,

wherein, in each set, the at least two fluorescent detection molecules carry a different fluorophore type, so that the Target Sequence TSi will eventually be characterized by at least two different tags, at least two different TCS and at least two different fluorophore types.

Thus, in this preferred embodiment, the mediator probes are able to directly bind to the TS, and, when bound during the amplification reaction, can release a tag that is complementary to a tag complementary sequence (TCS) located on a fluorescent detection molecule. The said fluorescent detection molecule carries a quencher group and a fluorophore, whose fluorescence is modified as a result of the hybridization or of the elongation of the tag sequence of the mediator probes, once released, on the TCS on said detection molecule.

In practice, when a partition contains the target sequence of interest, the PCR reaction will trigger an increase in the fluorescence intensity of the at least two fluorophore types carried by the fluorescent detection molecules, or "reporters". Based on the measured fluorescence intensity of each fluorophore type, each partition can be classified as negative or positive using a similar methodology as described above using TaqMan® probes.

In the method of the invention, the fluorescent detection molecule can be any universal reporter classically associated with a mediator probe. It is possible to use in particular any linear or beacon universal reporter containing a tag complementary sequence, a fluorophore type and a quencher. If the fluorescent detection molecule contains a loop, the Tag Complementary Sequence TCS can be located within the loop ([11]) or outside the loop (cf. EP 2776585).

In a preferred embodiment, the fluorescent detection molecules used in the method of the invention are any reporter probes carrying at least a fluorophore type, a quencher and a TCS, for example Molecular Beacons, Scorpions, HybProbes, Hybeacons and any other tools that have been successfully established to be used as universal reporter (see for example on https://www.biomers.net/en/Products/DNA/Mediator_Probe_PCR.html). In a particularly preferred embodiment, the fluorescent detection molecules used in the method of the invention are molecular beacons or complementary single-stranded fluorescent detection reporter (CSSFR) molecules, as will be now described in more details.

Molecular beacon molecules are well-known in the art. They are for example described in EP2776585, in [11], etc.). These molecules are specifically designed to display a nucleotide hairpin structure, coupled to a quencher and a fluorophore type.

In these reporter molecules, the Tag Complementary Sequence (TCS) can be either present in the hairpin structure, or outside the hairpin structure.

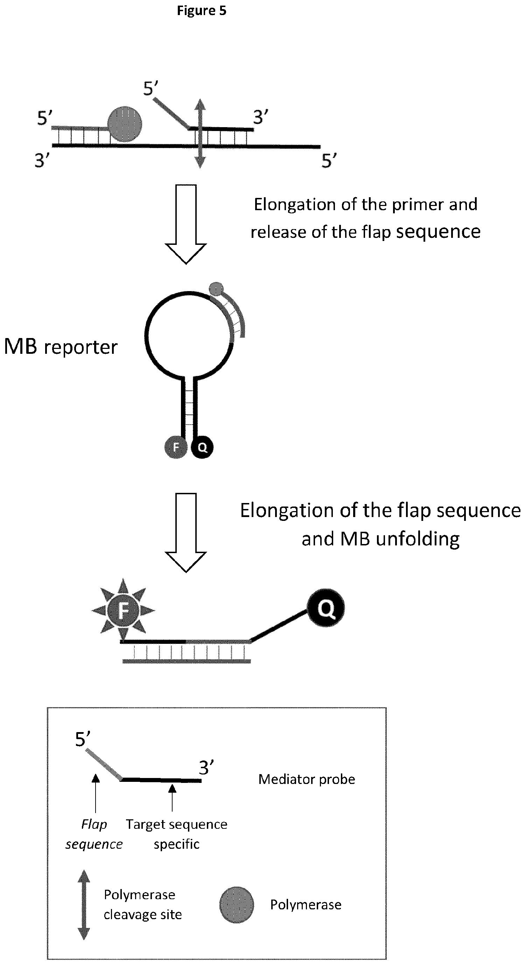

The present inventors have successfully tested the method of the invention by using four molecular beacon reporters for measuring the concentration of six Target Sequences (figures 5-6, example 2A). In this example, the TCS were located in the hairpin structure of the molecular beacon (figures 5-6).

In practice, it can be difficult to generate molecular beacon molecules that have 3D constraints due to their hairpin structure. Moreover, a molecular beacon reporter molecule should be synthetised by grafting the fluorophore type and the quencher on the same strand, what is sometimes tricky with some fluorophore types and more costly than reporter molecules with only either a fluorophore or quenchers. For these reasons, the inventors proposed to use CSSFR as alternative mediator probes.

Mediator probes / Complementary Single Stranded Fluorescent Reporters (CSSFR)

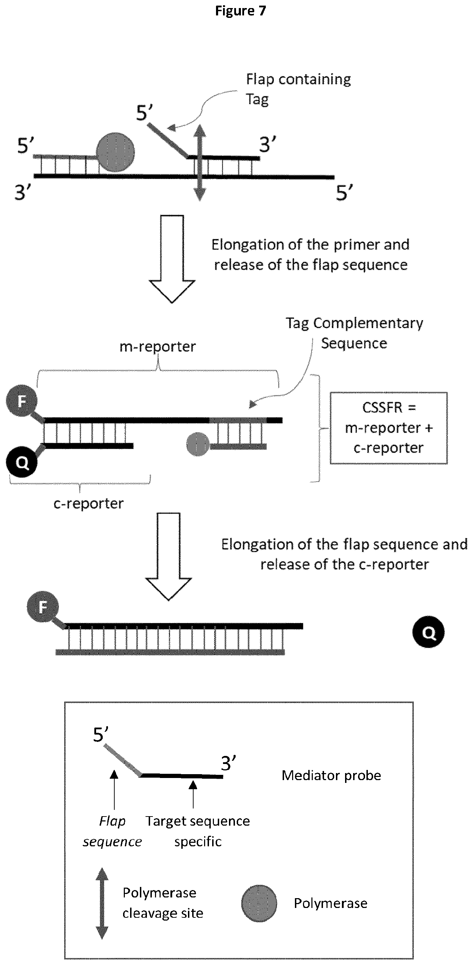

The Complementary Single Stranded Fluorescent Reporters contain the following oligonucleotides (see figure 7):

- a main reporter oligonucleotide molecule (m-reporter) containing:

  i) a sequence that is complementary to the tag sequence ("tag complementary sequence", TCS) carried by the corresponding mediator probe, and
  ii) at its 5' end, a reporter molecule which may be either a fluorophore or a quencher group;

- a complement reporter oligonucleotide molecule (c-reporter) containing:

  i) a sequence that is complementary to the 5' end sequence of the m-reporter;
  ii) at its 3' or 5' end, a reporter molecule which may be a fluorophore group in case the m-reporter has a quencher group, or a quencher group in case the m-reporter has a fluorophore group.

In another embodiment, the c-reporter can be modified at both the 3' and 5' ends to add one quencher and one fluorophore group, or 2 fluorophore groups, or 2 quencher groups.

In this system, the fluorescence of the fluorophore group is modified as a result of the hybridization of, or of the elongation of, the tag sequence of the 5' terminal mediator region, once cleaved from the mediator probe, on the tag complementary sequence (TCS) on the m-reporter.

The inventors successfully tested the method of the invention by using four CSSFR molecules for measuring the concentration of six Target Sequences (figure 7, example 2B).

Using universal reporters, and in particular CSSFRs instead of TaqMan® probes present a number of advantages:

First, the "color combination" multiplexing approach can be implemented using only a small number of universal reporters. When using $M$ detection channels, only $M$ universal reporters would be required to implement color combination. This remains true irrespectively of the number of fluorophore types combined for each target.

For example, using 6 detection channels and 2 fluorophore types per target sequences, only 6 CSSFR types will be required to detect up to 15 different targets. And, for each target sequences, only 2 different mediator probes should be designed, each associated to a different CSSFR.

By using the 6 following CSSFR molecules:

- CSSFR#1-FAM: containing TCS#1 and fluorophore type FAM
- CSSFR#2-YY: containing TCS#2 and fluorophore type YY
- CSSFR#3-ATTO550: containing TCS#3 and fluorophore type ATTO550
- CSSFR#4-ROX: containing TCS#4 and fluorophore type ROX
- CSSFR#5-Cy5: containing TCS#5 and fluorophore type Cy5
- CSSFR#6-ATTO700: containing TCS#6 and fluorophore type ATTO700

If the Target Sequence $TS_1$ is detected using:

- Mediator Probe #1: containing a TSi specific sequence + tag #1 (triggering CSSFR#1)
- Mediator Probe #2: containing the same TSi specific sequence as #1 + tag #2 (triggering CSSFR#2)

If the Target Sequence $TS_2$ is detected using:

- Mediator Probe #3: containing $TS_2$ specific sequence + tag #1 (triggering CSSFR#1)
- Mediator Probe #4: containing the same $TS_2$ specific sequence as #3 + tag #3 (triggering CSSFR#3)

If the Target Sequence $TS_3$ is detected using:

◦ Mediator Probe #5: containing TS$_3$ specific sequence + tag #1 (triggering CSSFR#1)
◦ Mediator Probe #6: containing the same TS$_3$ specific sequence as #5 + tag #4 (triggering CSSFR#4)

If the Target Sequence TS$_4$ is detected using:

◦ Mediator Probe #7: containing TS$_4$ specific sequence + tag #1 (triggering CSSFR#1)
◦ Mediator Probe #8: containing the same TS$_4$ specific sequence as #7 + tag #5 (triggering CSSFR#5)
◦ ...

The table below compares the number of TaqMan® probe types and the number of CSSFR types required to reach a given level of multiplexing for different numbers of detection channels using color combination with 2 colors per target sequences.

| Number of detection channels | Level of multiplexing | Number of different TaqMan® probe types | Number of CSSFR types | Number of mediator probe types |
|---|---|---|---|---|
| 2 | 1 | 2 | 2 | 2 |
| 3 | 3 | 6 | 3 | 6 |
| 4 | 6 | 12 | 4 | 12 |
| 5 | 10 | 20 | 5 | 20 |
| 6 | 15 | 30 | 6 | 30 |
| 7 | 21 | 42 | 7 | 42 |

Another advantage of using universal reporters instead of TaqMan® probes is a reduction in the development time and costs of the assays. Indeed, fluorescent-labeled oligonucleotides like TaqMan® probes or the fluorescent reporter molecules typically take more time to be manufactured than bare oligonucleotides sequences like PCR primers or mediator probes. Typically, PCR primers or mediator probes can be ordered and supplied in less than 1 week while fluorescent-labeled oligonucleotides can take up more than 1 month to be supplied from ordinary oligonucleotide manufacturers. Similarly, the cost of fluorescent-labeled oligonucleotides is usually 5 to 10 times higher than the cost of an equivalent primer sequence. Thus, the less fluorescently-labelled molecules is needed, the better it will be in terms of costs and development time.

Another advantage of using universal reporters is that the same reporters can be used across different assays targeting different types of sequences or mutations. These assays would differ from one another by the types of mediator probes which would have different 3' target specific sequences but would share the same set of reporter tag sequences. By sharing reporters across different assays, the reporter fluorescent-labeled oligonucleotides can be ordered in larger quantities, leading to further reductions in costs by economies of scale.

Finally, another advantage of the universal reporter approach for "color combination" multiplexing, compared to TaqMan®- based approaches, is that the optimization of the ratio of the reporters and of the compensation matrix only has to be done once and does not change during assay development and optimization of the mediator probe sequences. Indeed, as discussed in the section regarding TaqMan® probes, the ratio of concentration between two competing TaqMan® probes has to be adjusted for each probe sequence. As a result, each time a probe sequence is changed during the assay development process, the ratio may need to be adjusted. Furthermore, a change in the TaqMan® probe sequence may lead to a modification of the compensation matrix as well, which would then need to be re-adjusted during the assay development process. Such adjustments require the collection of additional experiment data and can be tedious when using more than 3 detection channels. By contrast, when using reporters for "color combination" according to the invention, the sequences do not change during the assay development process. Assay development typically involves optimization on the primers and on the target -specific mediator probe sequences, none of which are fluorescently-labeled.

Abundant WT on its own channel, 1 color only.

As will be further detailed below in the statistical section, in certain embodiments the invention provides that a given first target sequence, which abundance is significantly higher than that of the other target species in the analyzed sample (typically, the abundant target species is the Wild Type), is color-coded using a first set of fluorophore types, while all other target sequence are color coded using a tow or more fluorophore types each selected from a second set of fluorophore types, all distinct from the first set.

Thus, in a preferred embodiment of the invention, no more than one fluorophore type is dedicated to the most abundant target sequence of the sample (typically, the abundant target sequence is the Wild Type).

In this case, as a unique fluorophore will be associated to a particular sequence of interest, there will remain n-1 fluorophore types that can be used for detecting the other Target Sequences. For example, if the number $n$ of fluorescence detection channels of the dPCR instrument is 6, the method of the invention will be performed by assigning to each TSi a unique combination of two out of 5 fluorophores types, i.e., it will be possible to detect $C_5^2 =$ 10 different TS$_i$, in addition to the WT sequence. And if the number $n$ of fluorescence detection channels of the dPCR instrument is 7, the method of the invention will be performed by assigning to each TSi a unique combination of two out of 6 fluorophores types, i.e., $C_6^2 = 15$ different TS$_i$, in addition to the WT sequence.

Embodiment employing Large Stokes Shift dyes.

In the prior art approaches to detecting fluorescence dyes useful for digital PCR approaches, the fluorophores are assigned to a "channel", which in fact corresponds in the fluorescence reader to an excitation source, e.g. a led and a filter, coupled to an emission filter. These emission / excitation filter pairs are designed according to the emission and excitation spectra of commonly used fluorophores, such as FAM, HEX, ROX, Cy® dyes, Atto™ dyes, and Yakima Yellow. In the fluorescence reader described in WO2022/063845, for example, six pairs of emission led and filters / excitation filters are described, which define six useable channels.

In an embodiment of the present invention, additional encoding channels are made available by the use of Large Stokes Shift Dyes (LSSD), and by accordingly decoupling the prior art emission / excitation pairs ([12], [13]). In other words, whereas the prior art typically refers to channels which comprise fixed emission/excitation filter pairs, the use of LSSD in this embodiment of the invention requires that the fluorescence reader be capable of exciting the LSSD in a first channel while detecting its fluorescence emission in a remote, distinct channel. This approach implies that the fluorescence detecting hardware be capable of selecting excitation and emission hardware independently from each other, and not as fixed pairs.

In a preferred embodiment of the invention, at least one of the fluorophore types is one of the Large Stokes Shift Dyes (LSSD).

In this embodiment of the present invention, the color combination may thus be effected with the use of one or more LSSD(s), in addition to the prior art commonly used fluorophores. Because the multiplexing capacity of the approach of the invention increases according to

$$C_n^k = \frac{n!}{k!(n-k)!}$$

, where $k$ is the number of colors used per target and $n$ is the total number of fluorescence detection channels, any increase in $n$ will substantially increase the multiplexing capacity of a given dPCR system. For example, using a dPCR system with 6 channels and encoding each target using two colors allows to multiplex up to 15 targets, while the addition of one LSSD, when using a dPCR system with 6 channels which has uncoupled excitation and emission capabilities, will allow to multiplex up to 21 targets.

The skilled artisan will know how to select LSSD for a particular application on a particular system. Considerations such as spectral separability from existing fluorophores based on their fluorescence characteristics, as well as charges carried by the LSSD molecules which may interfere with DNA or the chemical system used by a particular dPCR instrument are to be taken into account.

In a non-limiting example, the present inventors have for example successfully implemented experimental protocols employing the following LSSDs:

| Fluorophore name | DyLight™-LS515 | DyLight™-LS510 | DY-521-XL |
|---|---|---|---|
| Tested configuration | Teal→Yellow (TY) , or Teal→Red (TR) Testedin TR Not tested inTY | Teal → Green (TG) Not tested | TR Testing underway |
| Supplier / Probe synthesis | Thermofisher Scientific / Conjugated in house | Thermofisher Scientific / Conjugated in house | Dyomics / Synthesised & Conjugated at Eurogentec |

In the method of the invention, it is possible to use any of these LSSDs (DyLight™-LS-515; DyLight™-LS510; DY-521-XL), as well as any other LSSD that can be conjugated to an oligonucleotide sequence like a TaqMan® probe.

In a more preferred embodiment, in the method of the invention, no more than one fluorophore type is dedicated to the most abundant species of the sample and at least one of the fluorophore type is one of the Large Stokes Shift Dyes (LSSD).

Statistical analysis of the data processing protocol of the invention

When color-combination is used to detect Target Sequences, partitions containing multiple targets (co-encapsulations) are ambiguous and must be discarded from the analysis. For example, an assay is set up to detect Target 1 using FAM & Cy3, Target 2 using FAM & Cy5 and Target 3 using Cy3 & Cy5 fluorophores. In such an example, a partition that is positive for FAM, Cy3 and Cy5 can be the result of co-encapsulation of Target 1 and Target 2, or Target 1 and Target 3, or Target 2 and Target 3, or of Target 1, Target 2 and Target 3. Consequently, when a partition is classified a FAM, Cy3 and Cy5 positive experimentally, it is impossible to make a prediction with regards to which Target Sequences are present in the partition. Such triple positive partitions are positive but ambiguous with regards to their content.
A consequence of discarding ambiguous partitions is a loss in the assay sensitivity.
The loss of sensitivity is directly proportional to the number of all excluded droplets relative to the maximum analyzable number of droplets.
Because during the analysis of a given target $i$, all the partitions which are positive for any other target $j$ are excluded, it can be shown that for said target i, the loss in sensitivity is:

$$\Delta_{Target_i}^{Sensivity} = 1 - e^{-\frac{v}{d}\Sigma_{j \neq i} c_j}$$

where v is the droplet volume, d is the dilution factor, $c_k$ the concentration for target $k$, $i$ the indicia for the target under study and j the indicia for all other targets.
In addition, since the error of a digital PCR result scales as $1/\sqrt{(V_{analyzed})}$ (see for example [14]), the error in the measurement (loss of precision) for a given target i increases by $e^{\frac{1}{2}\frac{v}{d}\Sigma_{j \neq i} c_j}$ .

From the above, it can be seen that the loss in sensitivity and precision depends on experimental factors only:

$$\sum_{j \neq i} v c_j$$

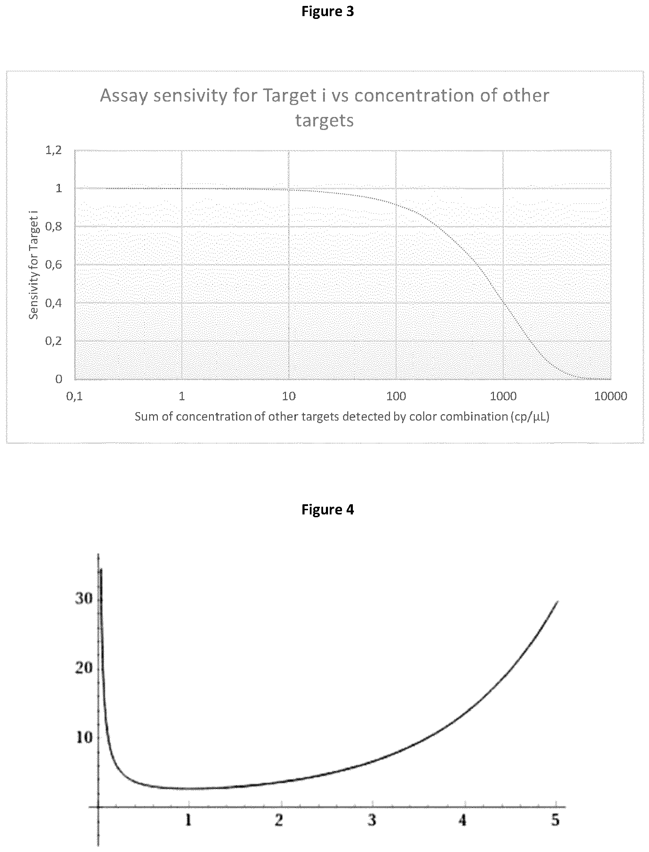

This is illustrated in Figure 3 where a theoretical calculation provides the sensitivity for Target i as a function of the sum of the concentrations of the other targets in the assay, using a Sapphire chip (Stilla Technologies, 1, Mail du Professeur Georges Mathé - 94800 Villejuif, France). Based on this graph, we can see that there is a sharp loss in sensitivity between 100 cp/$\mu$L and 1000 cp/$\mu$L.
It is indeed the case that as droplets with more than one target sequence are discarded from the analysis, the content of these droplets is not analyzed. This loss in analyzed droplets impacts directly the assay sensitivity. The more co-encapsulations, the more impacted the sensitivity is. Figure 3 shows a model of encapsulation events for different background genes concentrations and how sensitivity is impacted with the background gene concentration level. For background levels below 100 cp/$\mu$l, the loss in sensitivity is less than 10% and it increases sharply for larger background levels.
According to the present invention, when the analyzed sample is of the liquid biopsy type, 80% of samples will have wild-type ctDNA concentrations in the PCR mix that will be below 200 cp/$\mu$L and mutant ctDNA concentrations 10-fold lower. Consequently, according to the present invention, using color combination to detect the wild-type DNA, one would expect to lose at most 20% of sensitivity in 80% of cases. For this reason, the color combination method according to the invention works very well for rare event detection applications, where the expected concentrations are low, thus leading to only a marginal loss of sensitivity.
Color combination according to the invention also works very well in assays where only one target is detected in a given sample, i.e., when the presence of one given target is most often associated with the absence of all other targets. Indeed, in this case, $\Sigma_{j \neq i} v c_j = 0$ and there is no loss of sensitivity.

Relative sensitivity calculations for the method of the invention in the context of Mutant Allelic Fraction determination

The color combination approach of the invention has an impact on the absolute sensitivity of an assay. Assuming an assay and a digital PCR system with perfect analytical sensitivity, meaning that if a detectable target is present in a partition, said partition will always be called as positive, then the limit of detection (LOD) with a 95% confidence level of the assay for each target i using the color combination approach will be:

$$LOD_{target_i} = \frac{3}{V_0} e^{\frac{v}{d} \Sigma_{j \neq i} c_j},$$

where $V_0$ is the volume of all partitions in the digital PCR experiment.

This means that there is a 95% confidence of detecting at least 1 positive partition and calling the sample as "positive" for target i if the target average concentration is at least greater than 3 copies in the analyzed volume

$$V_0 * e^{-\frac{v}{d} \Sigma_{j \neq i} c_j}.$$

In some applications, in particular in liquid biopsy, the most important sensitivity metric is not absolute sensitivity but sensitivity relative to a reference template, e.g. Wild-Type (WT) DNA.

In such a case, the metric of interest is the Mutant Allelic Fraction (MAF):

$$MAF = \frac{c_{mut}}{c_{WT}}$$

The lowest detectable concentration of the mutant target is the $LOD_{target_i}$ calculated above, which depends on the concentration of the WT DNA (the concentration of the wild-type target is included in the sum of concentrations of all other targets $\Sigma_{j \neq i} c_j$).

Assuming that the WT DNA concentration is significantly higher than the concentration of any other target in the assay, the lowest measurable MAF using color combination is:

$$MAF_{min}(c_{WT}) = \frac{LOD_{mut}}{c_{WT}} = \frac{\frac{3}{V_0} e^{\frac{v}{d} \Sigma_{j \neq i} c_j}}{c_{WT}} \approx \frac{3}{V_0} \frac{e^{v\, c_{WT}}}{c_{WT}} = \frac{3}{N} \frac{e^{v\, c_{WT}}}{v c_{WT}}$$

assuming the dilution factor d = 1 and $\Sigma_{j \neq i} c_j \approx c_{WT}$.

As a next step, one can write:

$$MAF_{min}(x) = \frac{3}{N} \frac{e^x}{x}$$

where $x = v\, c_{WT}$ becomes the average droplet occupancy of the wild-type DNA (average number of wild-type DNA molecules in each droplet).

Figure 4 shows a plot of the function f(x) = $e^x$ / x. One can see that the lowest MAF measurement possible is obtained when $x = v\, c_{WT}$ = 1, leading to $MAF_{min}$ = 3 e/N.

By comparison, in the context of the prior art approach where one fluorophore type is used for each target sequence in the design of the assay, the lowest MAF measurement possible is when the WT concentration is highest,

$$c_{WT} \approx \frac{N \ln(N)}{V_0} \qquad MAF_{min} = \frac{3}{N \ln(N)}$$

i.e., at saturation. This occurs when ⟨above⟩, leading to the lowest ⟨above⟩.

As such, when using the method according to the invention with a digital system capable of generating N=20 000 partitions, the lowest measurable MAF with a given assay is ln(20 000)*e = 27 times higher than when using the prior art simple "1 color = 1 target" assay.

To put this into perspective, using the method according to the invention, the lowest measurable MAF would be 0.03% for a Sapphire chip (Stilla Technologies, 1, Mail du Professeur Georges Mathé - 94800 Villejuif, France) and 0.05% for an Opal chip (Stilla Technologies, 1, Mail du Professeur Georges Mathé - 94800 Villejuif, France). These numbers remain acceptable, given that typically reported MAFs with clinical utility are above 0.01%.

Alternatively, one can compare the "1 color = 1 target" prior art approach and the "color combination" method according to the invention by comparing $MAF_{min}$ for a given $x = v\,c_{WT}$:

$$\frac{MAF_{min}^{color\ comb}}{MAF_{min}^{simple}} = \frac{3}{N}\frac{e^x}{x}\frac{xN}{3} = e^x$$

Thus, the loss in relative sensitivity increases as the WT concentration increases.

For example, on a Sapphire chip (Stilla Technologies, 1, Mail du Professeur Georges Mathé - 94800 Villejuif, France), if $c_{WT}$ = 1000 $cp/\mu L$ (corresponding to x=0.62), the relative sensitivity loss is $e^{0.62}$ = 1.86 and the minimum achievable MAF would be 0.44% in the color combination method according to the invention *versus* 0.24% with the simple assay design of the prior art.

Statistical analysis also shows that an additional target can be detected on top of the 15 using only one of the colors used for color combination without any major loss in performance.

In this approach, we detect a first Target Sequence $TS_e$ using only one fluorophore type. The concentration of $TS_e$ is note $C_e$. All other target sequences TSi are detected using two fluorophore types.

The concentration $C_e$ can be measured by counting the partitions that are either all negative and those that are only positive for the fluorophore type used for $TS_e$. This means that all partitions that are positive for more than one flurophore type are excluded from the concentration measurement of $TS_e$. The loss of sensitivity for $TS_e$ is consequently

$$\Delta_{TS_e}^{Sensivity} = 1 - e^{-\frac{v}{d}\Sigma_i c_i}$$

The sum of the concentration of all targets other than $TS_e$ is the main parameter driving loss of sensitivity, in the same manner than for the targets that are detected using 2 different fluorophore types.

In case where one target sequence is expected to have a high concentration, such as a wild-type sequence, it can be preferable to detect this such abundant target sequence using a distinct and separate fluorophore type (e.g. FAM/blue channel), while using the other fluorophore types and detection channels to detect low concentration target sequences (such as mutant target sequences) using color combination. With this approach, the sensitivity of the assay for the low concentration target sequences is not impacted and reduced by the present of the abundant isolated target sequence.

Generating a fluorescence compensation matrix in the context of target sequences encoded using the method of the invention

In a preferred embodiment, the method of the invention contains, prior to step a), a step of generating a fluorescence compensation matrix, as described above, said fluorescence compensation matrix being then applied to measure, for each partition, the fluorescence levels or signal intensity from the *M* fluorophore types using the *n* signals from the *n* fluorescence detection channels.

In particular, a compensation matrix is built in which, for each fluorescent probe used in the assay, fluorescence parameters are defined for each fluorescence channels.

In the prior art methods, said compensation matrix was built by collecting fluorescence data from samples made of monocolor controls for all fluorescent probes present in the assay. In this case, the monocolor control for a given fluorescent probe consisted in a mixture of all the primers and all fluorescent probes used in the assay with the addition of the DNA template for the target sequence detected by the fluorescent probe of interest.

By contrast, in the methods of the invention where target sequences are encoded using color combination, the generation of the fluorescence compensation matrix requires a different setup: in this embodiment of the method of the invention, the mixture of primers and fluorescent probes are in the reaction mix as in the prior art methods, except that one of the fluorescent probes used for color encoding the target sequence under study is missing. For example, if the $TS_1$ is detected by the following two probes: one in green and one in yellow, then the mix for the monocolor control for green would not contain the yellow probe for target $TS_1$, therefore it is ensured that $TS_1$ is detected by only the one probe of interest (the green) in this special case used as a reference for the compensation matrix.

Computer implementation of the data analysis method of the invention

Each step d) and/or e) and/or f) of the method according to the invention previously described is not performed in a purely abstract or purely intellectual manner but involves use of a technical means.

Typically, step d) of the method according to the invention previously described should be implemented by an optical detector (for measuring, for each partition, the intensity of fluorescence for each fluorophore type), and by at least one computer, one central processing or computing unit, one analogue electronic circuit (preferably dedicated), one digital electronic circuit (preferably dedicated) and/or one microprocessor (preferably dedicated). Moreover, software means are useful for setting a positivity threshold intensity for each fluorophore type.

Typically, each e) and/or f) of the method according to the invention previously described can be implemented by at least one computer, one central processing or computing unit, one analogue electronic circuit (preferably dedicated), one digital electronic circuit (preferably dedicated) and/or one microprocessor (preferably dedicated) and/or by software means.

In another aspect, the present invention concerns a computer program comprising instructions which, when executed by a computer, implement the steps e) and f) of the method according to the invention (and preferably also at least a part of step d) of the method according to the invention, in particular setting a positivity threshold intensity for each fluorophore type).

In another aspect, the present invention concerns a computer program product comprising instructions which, when the program is executed by a computer, cause the computer to carry out the steps e) and f) of the method according to the invention (and preferably also at least a part of step d) of the method according to the invention, in particular setting a positivity threshold intensity for each fluorophore type).

In another aspect, the present invention concerns a computer-readable storage medium comprising instructions which, when executed by a computer, cause the computer to carry out the steps e) and f) of the method according to the invention (and preferably also at least a part of step d) of the method according to the invention, in particular setting a positivity threshold intensity for each fluorophore type).

Advantages over the dPCR assays of the prior art

As mentioned above, the "intensity-based multiplexing" of Lindner et al, 2021 ([4]) combines, for at least one of the fluorophore type used in the assays, two or more TaqMan® probe types that share the same fluorophore type but which have different target sequences. The two or more TaqMan® probe types that share the same fluorophore type are combined in the assay at two distinct concentrations. By using only 2 probe types per fluorophore type, one probe type would have a low concentration ("lo" type) and one probe type would have a high concentration ("hi" type). As a result, after PCR, if a partition contains the target for the "lo" type, the fluorescence level of the associated fluorophore type in the partition would be above a set positivity threshold but at a lower level than if the partition would contain the target for the "hi" type. After PCR, assuming that all targets are present in the sample, for each fluorophore type for which intensity-based multiplexing has been implemented, there would be negative population of droplets, a first "lo" positive population of droplets, a second "hi" positive population of droplets with a higher measured fluorescence level and possibly a third "lo+hi" positive population of droplets with an even higher measured fluorescence level (these would contain both the "lo" and "hi" targets such that the fluorescence signals add up). Using this approach, the different targets are distinguished by exploiting the measured fluorescence intensity of the partitions, in addition to an initial binary classification of the droplets according to set positivity thresholds.

Assuming that 2 levels of fluorescence intensity are used for each M=N fluorescence detection channels/fluorophore types, the maximum number of targets that are detectable, i.e., the maximum level of multiplexing, would be of maximum 2N. Using 6 detection channels, this leads to a maximum of 12 targets.

When compared to the "color combination" approach of the invention, the maximum level of multiplexing with intensity-based multiplexing is thus lower as soon as the number of fluorescence channels exceeds 5, and is rapidly much lower. For example, with n=10 fluorescence detection channels, the color combination approach of the invention allows for up to 45 targets to be detected, while the intensity-based multiplexing of Lindner et al. only allows for less than half, at a maximum of 20 targets.

| Number of detection channels | 2-levels intensity-based multiplexing (Lindner et al)<br>Max level of multiplexing | 2-color color combination of the invention<br>Max level of multiplexing |
|---|---|---|
| 2 | 4 | 1 |
| 3 | 6 | 3 |

(continued)

| Number of detection channels | 2-levels intensity-based multiplexing (Lindner et al) Max level of multiplexing | 2-color color combination of the invention Max level of multiplexing |
|---|---|---|
| 4 | 8 | 6 |
| 5 | 10 | 10 |
| 6 | 12 | 15 |
| 7 | 14 | 21 |
| 8 | 16 | 28 |
| 9 | 18 | 36 |
| 10 | 20 | 45 |

Another drawback of the intensity-based approach of Lindner et al, compared to the color-combination of the invention, is the complexity of the droplet classification and of the data analysis. Using the color-combination of the invention, only one positivity threshold needs to be set for each fluorophore type used in the experiment. Using intensity-based approaches, multiple thresholds must be set for each fluorophore type to distinguish between the multiple populations of positive partitions that all have distinct fluorescence intensities. When using a 2-level intensity-based approach, three thresholds are required for each fluorophore type used to appropriately classify the partition populations and appropriately detect and quantify the targets of interest: one threshold for "lo" positive partitions, one threshold for the "hi" positive partitions and one threshold for the "lo+hi" positive partitions.

The table below compares the number of thresholds for both approaches for different numbers of fluorescence detection channels:

| | 2-levels intensity-based multiplexing (Lindner et al) | | 2-color color combination (method of the invention) | |
|---|---|---|---|---|
| Number of detection channels | Max level of multiplexing | Number of thresholds | Max level of multiplexing | Number of thresholds |
| 2 | 4 | 6 | 1 | 2 |
| 3 | 6 | 9 | 3 | 3 |
| 4 | 8 | 12 | 6 | 4 |
| 5 | 10 | 15 | 10 | 5 |
| 6 | 12 | 18 | 15 | 6 |
| 7 | 14 | 21 | 21 | 7 |
| 8 | 16 | 24 | 28 | 8 |
| 9 | 18 | 27 | 36 | 9 |
| 10 | 20 | 30 | 45 | 10 |

This table highlights that the color-combination approach of the invention allows for higher levels of multiplexing with less thresholds and a simple data analysis.

In addition, when using intensity-based multiplexing, the classification of partitions using positivity thresholds will often fail due to interactions between the simultaneous PCR amplification of a target associated to one fluorophore type with a PCR amplification of a target associated to another fluorophore type. For example, if a partition contains a target associated with a "hi"-FAM TaqMan® probe type and also contains a target associated with a "hi"-Cy5 TaqMan® probe type, during PCR amplification, both targets will be amplified simultaneously. This simultaneous amplification can reduce the efficiency of one or both of the PCR reactions, such that at the end of the PCR amplification, the measured fluorescence intensity from the "hi"-FAM TaqMan® probe types may be lower than expected, and comparable to the expected fluorescence intensity of a "lo"-FAM TaqMan® probe. When analyzing the data using positivity thresholds described above, the partitions would be misclassified as containing the "lo"-FAM target, instead of the "hi"-FAM target, leading to an incorrect detection and quantification. This happens in particular when two or more TaqMan® probe types "compete" during the PCR amplification for the same set of PCR primers. To circumvent the problem, more complex approaches for the classification of the partitions have been used. They rely on the drawing of multiple multidimensional polygonal areas around all partitions that contain a given target of interest. While this polygonal classification approach remains practical with 2 or 3 fluorescence detection channels (2D or 3D polygons), it becomes very cumbersome for higher numbers of detection channels.

Another drawback to intensity-based multiplexing approaches is that the classification of the partitions is not robust against the phenomenon of "rain" in digital PCR.

Ideally in digital PCR, all partitions that contain the same target (or targets) of interest would always be amplified with reaction efficiency and have the same measured fluorescence level. For a well-designed assays, there would be a clear difference in measured fluorescence level between a negative and a positive partition. Furthermore, the measured fluorescence level of a partition cannot be in between the level for negative and positive partitions as this would not correspond to either case of a negative or positive partition. Experimentally, however, partitions with intermediate levels of fluorescence do exist. They are usually partitions that contain a targeted nucleic acid molecule but for which the PCR reaction occurred with a reduced efficiency such that the end-point fluorescence level is below the usual fluorescence level for a positive partition. These partitions of intermediate levels in fluorescence intensity are called "rain".

The causes for "rain" are multiple:

- poor assay design;

- partial malfunction of the digital PCR system or the digital PCR consumable used:

    ○ thermal malfunction during PCR;
    ○ fluorescence readout error;
    ○ unusual partition volume;
    ○ dust particles;

- PCR inhibitors contained in the sample;

- sample-induced reduction in PCR efficiency.

Given the number of causes for rain, some level of rain is inevitable when performing digital PCR experiments.

When using a simple "1-color 1-target" approach in digital PCR, rain is either treated as positive partitions when the fluorescence level is above the set positivity threshold. Or rain is excluded from the analysis. However, when using an "intensity-based multiplexing" approach, rain causes additional problems of misclassification. Indeed, rain coming from partitions that should be with "hi"-levels of fluorescence intensity may end up having a fluorescence intensity that corresponds to partitions within the "lo"-levels of fluorescence intensity. In such cases, the partition would be misclassified as containing the target corresponding to the "lo"-level of fluorescence intensity, instead of being classified as containing the target corresponding to the "hi"-level of fluorescence intensity. Such misclassification of rain partitions is inevitable when using an "intensity-based multiplexing" approach and it can lead to false positive results and additional errors in the quantification of the targeted nucleic acids.

When using a "color-combination" approach according to the invention, there is only one positivity threshold set of each fluorophore type such that rain can be treated in the same manner as when using a simple "1-color 1-target" approach.

In conclusion, compared to "intensity-based multiplexing, the "color-combination" approach described herein has the following advantages:

- increased levels of multiplexing when the number of detection channels exceeds 5;

- simplified partition classification;

- significantly reduced number of positivity thresholds required to classify the partitions;

- improved robustness against rain.

DESCRIPTION OF THE FIGURES

Figure 1 - data from an experiment detecting 6 target sequences at a concentration of 100 cps/$\mu$L where the color combination uses 2 fluorophore types per target sequence and 4 fluorophore types in total across all 6 target sequences (example 1). Units on the x- and y-axis are Arbitrary Fluorescence Units (AFU) reflecting the fluorescence intensity of the fluorophore types.

Figure 2 - a) Dilution series of a single target sequence detected by color combination without any other target sequences. b) Dilution series of a single target sequence detected by color combination with a background of 5 other target sequences. c) Confidence interval on a target sequence (PhiX) at 100 cp/$\mu$l in an increasing background level of another target sequence (Lambda).

Figure 3 - Assay sensitivity of a target with increasing background levels.

Figure 4 - plot of the function $f(x) = e^x / x$.

Figure 5 - details the principle of combination of mediator probes and molecular beacons used as universal reporters.

Figure 6 - shows the principle of the combination mediator probes with molecular beacon as universal reporters A: Target Sequence 1; B: Target Sequence 2.

Figure 7 - details the principle of combination of mediator probes and CSSFR used as universal reporters.

Figure 8 - 1D thresholding in the universal reporter experiment using linear reporters. Units on the x-axis are the droplet indices, stacked for 8 samples; units on the y-axis are Arbitrary Fluorescence Units (AFU) reflecting the fluorescence intensity of the fluorophore types.

Figure 9 - data from an experiment detecting 3 target sequences at a concentration of 100 cps/$\mu$L where the color combination uses 3 fluorophore types per target sequence (example 3). Units on the x- and y-axis are Arbitrary Fluorescence Units (AFU) reflecting the fluorescence intensity of the fluorophore types.

Figure 10 shows data from a typical experiment detecting 11 target sequences at a concentration of 100 cps/$\mu$L (except ESR1 E380WT, used at ca. 200 cps/$\mu$L and for ALB where the concentration was ca. 160 cps/$\mu$L) using a total of 5 fluorescence types, where the color combination uses 2 fluorophore types per target sequence for 10 target sequences and 1 gene (de119ref) is visualized with only one fluorophore type (Cy3/Green) (see example 4).

Units on the x- and y-axis are Arbitrary Fluorescence Units (AFU) reflecting the fluorescence intensity of the fluorophore types.

EXAMPLES

EXAMPLE 1: Use of two TaqMan® probes per target sequence for effecting the color combination method of the invention: 6 target sequences - 2 colors per target sequence - 4 colors total

*Materials and Methods*

Forward and reverse primers for the different Target Sequences (see table below) are used at a concentration of 0.5 $\mu$M.

Two probes with different fluorophores and quenchers are used for each target sequence. The combination of target sequences and probes with dyes and the concentration used for those probes are detailed in the table.

The combination of fluorophore types/colors applied per target sequence is the following: ESR1 L536P (Infra-Red and Yellow), PhiX 174 (Infrared and Teal), PBR322 (Red and Infrared), ALB (Red and Teal), Lambda (Yellow and Teal) and puc18 (Yellow and Red).

| Name | Sequence | Dye | Quencher | Provider | Concentration (μM) |
|---|---|---|---|---|---|
| PhiX174 F | TCTTTCCAAGCAACAGCAG | | | Eurogentec | 0.5 |
| PhiX174 R | AATACTGACCAGCCGTTTGA | | | Eurogentec | 0.5 |
| PhiX174-ATTO700 | TCCGAGATTATGCGCCAAATGC | ATTO700 | BHQ3 | Eurogentec | 0.25 |
| Phix174-YY | TCCGAGATTATGCGCCAAATGC | Yakima Yellow | BHQ1 | Eurogentec | 0.25 |
| ESR1 530X F | CTGTACAGCATGAAGTGCAAG | | | Eurogentec | 0.5 |
| ESR1 530X R | GCATGTAGGCGGTGG | | | Eurogentec | 0.5 |
| ESR1 L536P-ATTO700 | TGCCCCCCTATGACCTGCT | ATTO700 | BHQ3 | IDT | 0.3 |
| ESR1 L536PROX | TGCCCCCCTATGACCTGCT | ROX | /Iowa Black RQ/ | IDT | 0.25 |
| Lambda F | GCCATTGTTTCTCTGTGGAG | | | Eurogentec | 0.5 |
| Lambda R | TCACGGTTCAGTTGTTCACC | | | Eurogentec | 0.5 |
| LambdaP-YY | TGATTGCCCGTCTCCGCT | Yakima Yellow | /ZEN/ /Iowa Black FQ/ | IDT | 0.25 |
| LambdaP-ROX | ACTGATTGCCCGTCTCCGCT | ROX | /Iowa Black RQ/ | IDT | 0.4 |
| pBR322 F | CGTTGATGCAATTTCTATGCG | | | Eurogentec | 0.5 |
| PBR322 R | TCGATAGTGGCTCCAAGTAG | | | Eurogentec | 0.5 |
| PBR322P-ATTO700 | CGCCCAGTCCTGCTCGCTTCG | ATTO700 | BHQ3 | Eurogentec | 0.25 |
| PBR322P-Cy5 | CGCCCAGTCCTGCTCGCTTCG | Cy5 | /TAO//low a Black RQ/ | IDT | 0.25 |
| ALB F | TGAAACATACGTTCCCAAAGAG TTT | | | Eurogentec | 0.5 |
| ALB R | CTCTCCTTCTCAGAAAGTGTGC ATAT | | | Eurogentec | 0.5 |
| ALB P-YY | TGCTGAAACATTCACCTTCCATG CA | Yakima Yellow | /ZEN/ /Iowa Black FQ/ | IDT | 0.25 |
| ALB P-Cy5 | TGCTGAAACATTCACCTTCCATG CA | Cy5 | BHQ2 | Eurogentec | 0.25 |
| pUC18 L1 F | CCTGCAGGTCGACTCTAG | | | Eurogentec | 0.5 |
| pUC18 L1 R | TGAGCGGATAACAATTTCACA | | | Eurogentec | 0.5 |
| pUC18 PROX | CCCGGGTACCGAGCTCGAATT | ROX | BHQ2 | Eurogentec | 0.25 |

(continued)

| Name | Sequence | Dye | Quencher | Provider | Concentration (μM) |
|---|---|---|---|---|---|
| pUC18 P-Cy5 | CCCGGGTACCGAGCTCGAATT | Cy5 | /TAO//low a Black RQ/ | IDT | 0.4 |

These sequences are displayed as SEQ ID NO: 1-24 in the sequence listing.

Supplier details are as follows:

- Kaneka Eurogentec SA - 5 Rue Bois Saint-Jean, 4102 Seraing, Belgium;
- IDT - Integrated DNA Technologies, Inc. - 1710 Commercial Park - Coralville, Iowa 52241 - USA.

Target sequences for the assay were double-stranded synthetic DNAs ("gBlocks™" fragments from Integrated DNA Technologies, Inc.) corresponding to the amplicons, used at an approximate concentration of 100 cps/$\mu$L.

To perform the PCR, naica® multiplex PCR MIX (Stilla Technologies, 1, Mail du Professeur Georges Mathé - 94800 Villejuif, France) with fluorescein was added to the reaction. To increase the stability of the components, DMSO at 4% (vol/vol) was included in the reaction. 7 $\mu$l of the reaction were loaded in an Opal chip (Stilla Technologies, 1, Mail du Professeur Georges Mathé - 94800 Villejuif, France). Negative controls (with no DNA) and monocolor controls were added to control the reaction and to build the matrix compensation.

Partition and PCR cycles are performed in a Geode instrument (Stilla Technologies, 1, Mail du Professeur Georges Mathé - 94800 Villejuif, France).

The cycling PCR program has one initial step of denaturation at 95°C for 3 minutes followed by 60 cycles of 95°C for 15 seconds and 62°C for 30 seconds as denaturation and hybridization-elongation temperatures, respectively.

The chip is then released from the pressure (returned to atmospheric pressure) at 25°C and at 5 mbar/s. The chip is imaged using Prism6 imager (Stilla Technologies, 1, Mail du Professeur Georges Mathé - 94800 Villejuif, France) using the following exposure times:

- for blue 125 ms;
- for teal 350 ms;
- for green 125 ms;
- for yellow 150ms;
- for red 500 ms; and
- for infrared 500 ms.

Data is analyzed using Crystal Miner software (Stilla Technologies, 1, Mail du Professeur Georges Mathé - 94800 Villejuif, France). For matrix compensation, monocolor controls are used.

The positivity threshold is set to identify negative and positive droplets from the rest of the droplets. This is typically done on 1D dot plot views.

In the next step, the population editor in the Crystal Miner software is used to define the populations for each target sequence. For example, for the Phix174 target sequence, positive droplets are designated as the ones positive for infrared and teal while the droplets displaying no fluorescence are counted as the negative droplets. Droplets displaying any other color combinations are excluded from the analysis. In this way, it is possible to retrieve the number of positive droplets for the target sequence and to estimate the concentration of said target sequence in the sample, among other characteristics.

Figure 1 shows data from a typical experiment detecting 6 target sequences at a concentration of 100 cps/$\mu$L using a total of 4 fluorophore types, where the color combination method of the invention uses 2 fluorophore types per target sequence.

*Results*

In order to assess the dynamic range accessible when detecting multiple target sequences encoded with 2 fluorophore types/colors, a series of experiments was carried out with the protocol detailed above.

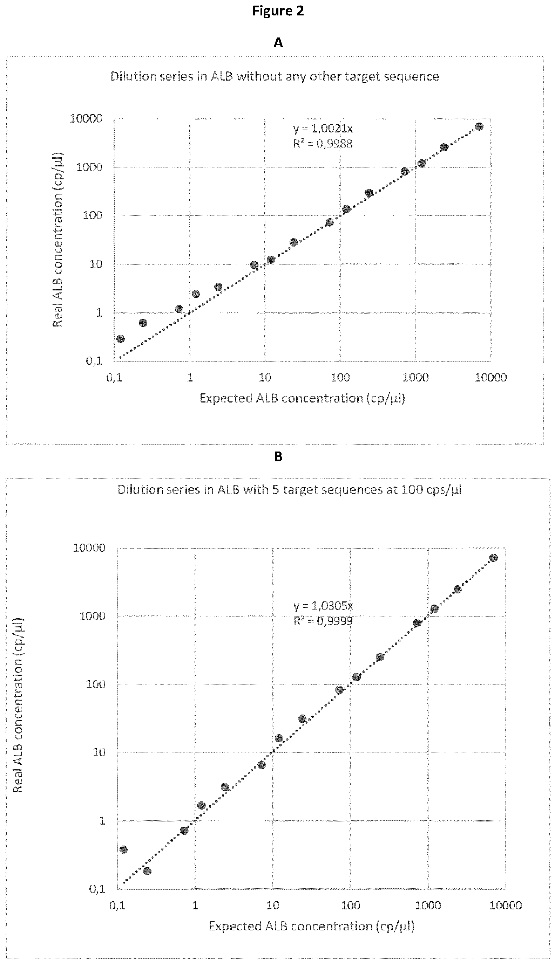

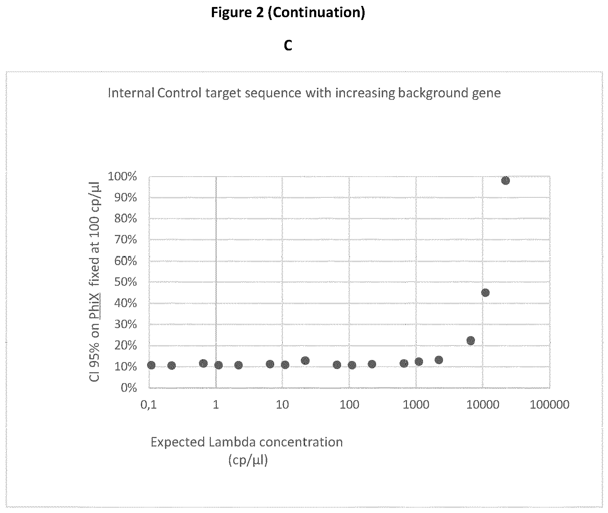

Good linearity between the expected and real concentrations was observed between 0.1 and 10,000 cp/$\mu$L, both when only 1 target sequence is added to the sample (Figure 2 a) and when 6 target sequences are detected together (Figure 2 b), in the latter case with one target sequence varying in concentration between 0.1 and 10,000 cp/$\mu$l while the other 5 target sequences are kept constant at 100 cp/$\mu$l. In addition, the measured Limit Of Detection values are comparable to those routinely obtained with the prior art method employing one color per target sequence, even despite the large background concentrations.

In yet another experiment, the confidence level for the detection of a target sequence at a fixed concentration was studied with varying amounts of a background target sequence (Figure 2 c). Here, the target sequence (PhiX) at a fixed concentration of 100 cp/$\mu$l was detected in the presence of increasing levels of a background target sequence (Lambda), from 0.1 cp/$\mu$l up to 10,000 cp/$\mu$l.

It can be seen that the confidence interval on the fixed concentration of PhiX remains stable around 10%, as expected, for concentrations of the background target sequence Lambda up to 2000 cp/$\mu$l, and subsequently increases

sharply for higher concentrations of the background target sequence.

This effect is believed to come from co-encapsulation events that are discarded from the analysis according to the method of the invention: the higher the background target sequence concentration, the higher the number of co-encapsulation events, and hence the lower the number of droplets analyzed according to the present method. A direct consequence is a lower confidence level on the fixed target sequence concentration when the background target sequence concentration (which could, for example, be the wild type) becomes too elevated.

As discussed in the foregoing, and in particular as illustrated in the section entitled *Statistical analysis of the data processing protocol of the invention,* whether this consequence of the method of the invention is a real limitation depends mostly on the application. For rare events detection in liquid biopsies, expected concentrations are low that this effect is not a factor. In situations where a reference target sequence or a highly concentrated target sequence is detected, one fluorophore type can be used in isolation to detect the highly concentrated target sequence to overcome the effect of co-encapsulation events.

EXAMPLE 2 : Use of two Mediator Probes (MP) triggering two universal reporters (UR) for each Target Sequence, to effect the color combination method of the invention

This approach consists in using a non-fluorescent probe (mediator probe, MP) composed of a sequence specific to the target and an artificial sequence called "flap" in 5' which is not complementary to the target. During elongation, the polymerase cleaves the probe after the first base hybridized to the target, thus releasing the flap sequence. This flap sequence in turn hybridizes to a universal reporter, which can for example be a molecular beacon (Example 2.A. below) or a linear reporter (Example 2.B. below).

A. Using molecular beacons as universal reporters

The use of molecular beacons as universal reporter is schematically depicted in Figure 5.

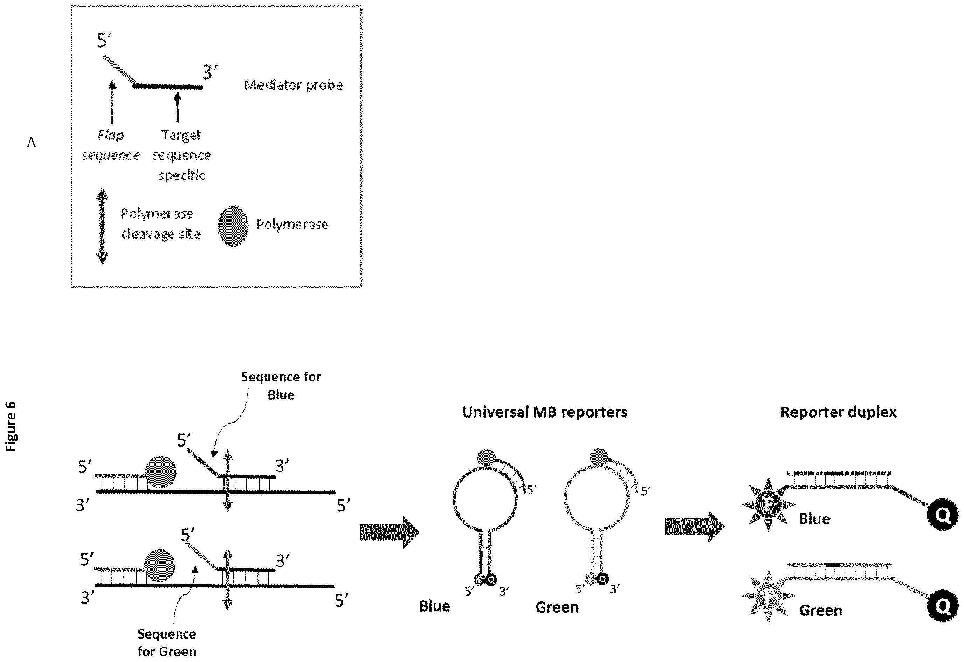

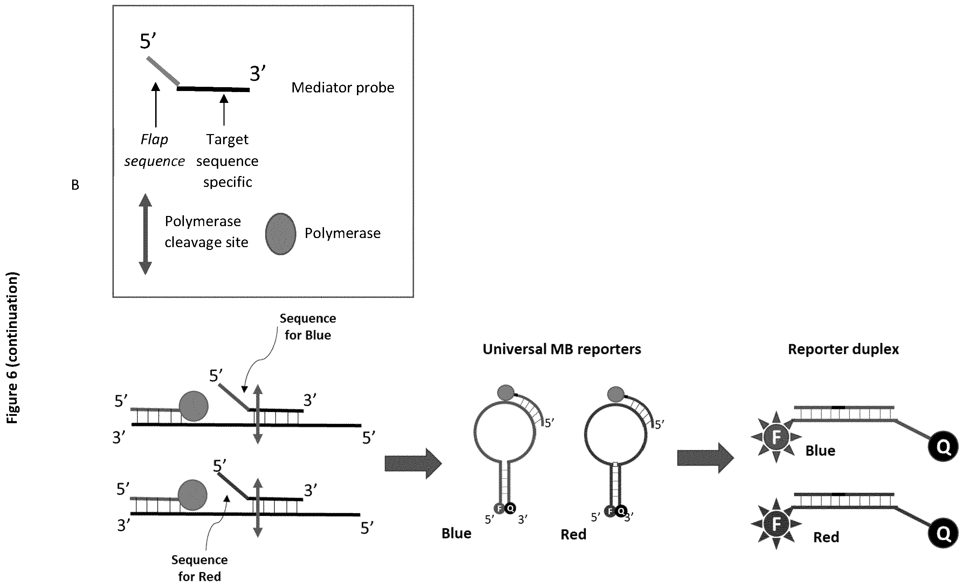

In this example, for each target sequence, two mediator probes allowing to activate two molecular beacons acting as universal reporters are in competition, as described in Figure 6. A total of 6 target sequences coded using 4 different fluorescence channels was followed.

*Materials and Methods*

For each target sequence, double-stranded synthetic DNAs ("gBlocks™" fragments from Integrated DNA Technologies, Inc) corresponding to the amplicons were used.

For color combination experiments, a mixture containing an estimated concentration of 1000 cp/μL (10x) of each DNA fragment was prepared.

The combination of colors applied per target sequence is the following: ESR1 L536P (Green and Red), PhiX 174 (Red and Teal), PBR322 (Green and Yellow), ALB (Red and Yellow), Lambda (Green and Teal) and puc 18 (Yellow and Teal).

All oligonucleotides were purchased from Kaneka Eurogentec SA (5 Rue Bois Saint-Jean, 4102 Seraing, Belgium). In this strategy, blockers are added to prevent the hybridization of the non-cleaved mediator probes to the molecular beacons which would result in an increase of the fluorescence background. In a preparatory step, primers, probes, molecular beacons and blockers corresponding to the 6 target sequences were assembled in a unique stock solution per reagent type. The composition of the reagent mixtures and the sequences of the oligonucleotides were as follows:

• The primer mix uses the primer sequences listed in the Table of Example 1.A. above at a concentration of 5 μM for each primer.

• The mediator probe mix was prepared with the following elements, which all contained a phosphate 3' modification (no 5' modification) and were mixed at 5 μM for each mediator probe:

| | |
|---|---|
| PhiXMP_TealMB | GTCTCGGTACTCTCTCCGAGATTATGCGCCAAATGCTTAC |
| LambdaMP_TealMB | CTCGGTACTCTCTGACTGATTGCCCGTCTCCGCT |
| pUC18MP_TealMB | TCGGTACTCTCTGACCCGGGTACCGAGCTCGAATT |
| LambdaMP_GreenMB | CGTGTGACTGATACTGATTGCCCGTCTCCGCT |
| pBRMP_GreenMB | CGTGTGACTGATACGCCCAGTCCTGCTCGCTTCG |
| ESR1MP_GreenMB | GTGTGACTGATACTGCCCCCCTATGACCTGCT |

(continued)

| pUC18MP_YellowMB | TCCTGCCTGCCCCGGGTACCGAGCTCGAATT |
| pBR322MP_YellowMB | TCCTGCCTGCCGCCCAGTCCTGCTCGCTTCG |
| AlbMP_YellowMB | CTGCCTGCCGTGCTGAAACATTCACCTTCCATGCA |
| ESR1MP_RedMB | CTCCGACCGGTGCCCCCCTATGACCTGCT |
| PhiXMP_RedMB | CTCCGACCGGTCCGAGATTATGCGCCAAATGCTTAC |
| AlbMP_RedMB | CTCCGACCGGTGCTGAAACATTCACCTTCCATGCA |

These sequences are listed as SEQ ID NO:25-36 in the sequence listing
• The blocker mix was prepared with the following elements, which all contained a phosphate 3' modification (no 5' modification) and were mixed at 5 $\mu$M for each blocker:

| PhiXBlocker_TealMB | ATAATCTCGGAGAGAGTACC |
| LambdaBlocker_TealMB | GGCAATCAGTCAGAGAGT |
| pUC18Blocker_TealMB | TACCCGGGTCAGAGA |
| LambdaBlocker_GreenMB | GGCAATCAGTATCAGTCA |
| pBRBlocker_GreenMB | GACTGGGCGTATCAGT |
| ESR1Blocker_GreenMB | GGGCAGTATCAGTC |
| pUC18Blocker_YellowMB | CCCGGGGCAGG |
| pBR322Blocker_YellowMB | TGGGCGGCAGG |
| AlbBlocker_YellowMB | GAATGTTTCAGCACGG |
| ESR1Blocker_RedMB | GGGGCACCGGT |
| PhiXBlocker_RedMB | ATCTCGGACCGGTC |
| AlbBlocker_RedMB | TTCAGCACCGGTC |

These sequences are listed as SEQ ID NO:37-48 in the sequence listing
• The molecular beacon mix was prepared with the following elements, which were mixed at 5 $\mu$M for each molecular beacon:

| Sequence name | Sequence | 5' modification | 3' modification |
|---|---|---|---|
| Teal_MB | CTCATCGCCAAGAATAGACAGTCAGAGAGTACCGAGACAGGCGATGAG | Yakima Yellow | BHQ®1 |
| Green_MB | CCTAGCCCAGACAAGAAGAGAACAGTATCAGTCACACGGGGCTAGG | Cy3 | BHQ®2 |
| Yellow_MB | CCGCGCCAGGCACGACCACAGGTGAGCACGGCAGGCAGGAGGGGGCGCGG | ROX | BHQ®2 |
| Red_MB | CGGCGCCAGCGGACGAGGCTGTGCACCGGTCGGAGGTGGGGGCGCCG | Cy5 | BHQ®2 |

These sequences are listed as SEQ ID NO:49-52 in the sequence listing

*PCR samples preparation*

The experiments were performed with an approximative concentration of the six target sequences at 100 cp/μL. A negative control without template (NTC, No Template Control) was also performed. For each condition two replicates were performed.

In addition to the DNA template mixture and the oligonucleotide mixtures described above, the naica® multiplex PCR MIX from Stilla Technologies (Stilla Technologies, 1, Mail du Professeur Georges Mathé - 94800 Villejuif, France) was used.

The detailed mix preparation for each of the two experimental conditions was as follows:

| Reagent | Stock solution concentration | Target mix 100 cp/μL | | No Template Control | |
|---|---|---|---|---|---|
| | | Final concentration | Volume (μL) | Final concentration | Volume (μL) |
| Water | | | 25,3 | | 30,8 |
| naica multiplex PCR MIX, buffer A | 10 | 1 | 5,5 | 1 | 5,5 |
| naica multiplex PCR MIX, buffer B | 100 | 4 | 2,2 | 4 | 2,2 |
| Primer mix | 5 | 0,5 | 5,5 | 0,5 | 5,5 |
| Mediator Probe mix | 5 | 0,5 | 5,5 | 0,5 | 5,5 |
| Molecular Beacon mix | 5 | 0,25 | 2,8 | 0,25 | 2,8 |
| Blocker mix | 5 | 0,25 | 2,8 | 0,25 | 2,8 |
| Target mix | 1000 | 100 | 5,5 | 0 | 0,0 |
| **Total** | | | **55,0** | | **55** |

*Chip loading and dPCR run*

The PCR samples were loaded in Sapphire chips (Stilla Technologies, 1, Mail du Professeur Georges Mathé - 94800 Villejuif, France) and processed in the Geode instrument according to the standard procedure. The usual Sapphire partitioning and release programs were used with the cycling PCR program described below:

- Initial denaturation: 3 min at 95°C;

- 60 cycles: 15 sec at 95°C then 30 sec at 62°C.

After the dPCR run, the chips were scanned using a Prism6 reader and the standard Sapphire scanning template (ScanningTemplate_Prism6_SapphireChip_naica-multiplex-PCR-MIX_Taqman_v1.4).

*Data analysis*

Data analysis was performed with the CrystalMiner software. To generate the compensation matrix, monocolor controls were used which consisted in the mix of the universal reporters in combination with one single mediator probe to activate only one color.

1D thresholds were set for each color channel to define negative and positive populations. Next, color assignment for each target sequence was set in the population editor section of the software. For example, PhiX174 was defined as the population positive in the teal and red channels and negative for all the other channels. After population edition, the results were exported and the quantification were reviewed. Experimentally measured concentrations were in good agreement with the theoretical concentration of 100 cp/μL for each of the six targets:

| phix concentration (cp/μL) | Lambda concentration(cp/μL) | pUC18 concentration (cp/μL) | pBR322 concentration (cp/μL) | ALB concentration (cp/μL) | ESR1concentration (cp/μL) |
|---|---|---|---|---|---|
| 95.6 | 98.4 | 95.0 | 105.0 | 131.5 | 92.1 |

B. Using linear reporters as universal reporters

The use of linear reporters as universal reporter is schematically depicted in Figure 7.

In this example, for each target sequence, two mediator probes allowing to activate two linear reporters acting as universal reporters are in competition. A total of 6 target sequences coded using 4 different fluorescence channels was followed.

*Materials and Methods*

For each target sequence, double-stranded synthetic DNAs ("gBlocks™" fragments from Integrated DNA Technologies, Inc) corresponding to the amplicons were used.

For color combination experiments, a mixture containing an estimated concentration of 1000 cp/μL (10x) of each DNA fragment was prepared.

The combination of colors applied per target sequence is the following: ESR1 L536P (InfraRed and Red), PhiX 174 (Red and Teal), PBR322 (InfraRed and Yellow), ALB (Red and Yellow), Lambda (InfraRed and Teal) and puc18 (Yellow and Teal).

All oligonucleotides were purchased from Kaneka Eurogentec SA (5 Rue Bois Saint-Jean, 4102 Seraing, Belgium). In a preparatory step, primers, probes, and linear reporters (comprising one A strand bearing the binding site for the flap sequence and the fluorophore; the other B strand, complementary to the A strand, and bearing the quencher) corresponding to the 6 target sequences were assembled in a unique stock solution per reagent type. The composition of the reagent mixtures and the sequences of the oligonucleotides were as follows:

• The primer mix uses the primer sequences listed in the Table of Example 1.A. above at a concentration of 5 μM for each primer.

• The mediator probe mix was prepared with the following elements, which all contained a phosphate 3' modification (no 5' modification) and were mixed at 5 μM for each mediator probe:

| | |
|---|---|
| PhiXMP_TealLR | GTCTCGGTACTCTCTCCGAGATTATGCGCCAAATGCTTAC |
| LambdaMP_TealLR | ACTCTCTCTTGGTCTACTGATTGCCCGTCTCCGCT |
| pUC18MP_TealLR | TCTCGGTACTCTCTCCCGGGTACCGAGCTCGAATT |
| pUC18MP_YellowLR | AGCTGCGTTGTATCCCGGGTACCGAGCTCGAATT |
| pBR322MP_YellowLR | AGCTGCGTTGTATCGCCCAGTCCTGCTCGCTTCG |
| AlbMP_YellowLR | CTAGCTGCGTTGTATGCTGAAACATTCACCTTCCATGCA |
| ESR1MP_RedLR | GTTGGATCGATGGTGCCCCCCTATGACCTGCT |
| PhiXMP_RedLR | GTTGGATCGATGGTCCGAGATTATGCGCCAAATGCTTAC |
| AlbMP_RedLR | GTTGGATCGATGGTGCTGAAACATTCACCTTCCATGCA |
| LambdaMP_InfraRedLR | GTACGATTGTGGTGACTGATTGCCCGTCTCCGCT |
| pBRMP_InfraRedLR | CGATTGTGGTGAGCGCCCAGTCCTGCTCGCTTCG |
| ESR1MP_InfraRedLR | GATTGTGGTGAGCTGCCCCCCTATGACCTGCT |

These sequences are listed as SEQ ID NO:53-64 in the sequence listing

• The A strand mix was prepared with the following elements, which contained the fluorophores indicated as 5' modifications (with no 3' modification), and mixed at 5 μM for each A strand:

| | | |
|---|---|---|
| Teal_LR | ATCGCCCAAGAATAGACAGAAGAATAGACCAAGAGAGAGTACCGAGAC | Yakima Yellow |
| Yellow_LR | ACATTGACATTGGACATTCAGACAGATAGATACAACGCAGCTAGGA | ROX |
| Red_LR | CGTATTGATTCATCTTCTCTCCTACACTGTACCATCGATCCAACTA | Cy5 |
| Infra-Red_LR | TCCATTGATAACCTTCTAATACTACAGCTCACCACAATCGTACTA | Atto700 |

These sequences are listed as SEQ ID NO:65-68 in the sequence listing

• The B strand mix was prepared with the following elements, , which contained the quenchers indicated as 3' modifications (with no 5' modification), and mixed at 5 $\mu$M for each B strand:

| | | |
|---|---|---|
| Teal_ABS | TATTCTTGGGCGAT | BHQ®1 |
| Yellow_ABS | TCCAATGTCAATGT | BHQ®2 |
| Red_ABS | AAGATGAATCAATACG | BHQ®2 |
| Infra-Red_ABS | AAGGTTATCAATGGA | BHQ®3 |

These sequences are listed as SEQ ID NO:69-72 in the sequence listing

*PCR samples preparation*

The experiments were performed at two linear reporter A strand concentration (0.25$\mu$M and 0.1$\mu$M) with an approximative concentration of the six targets at 100 cp/$\mu$L. For both linear reporter concentrations, negative control without template (NTC, No Template Control) were also performed. For each condition two replicates were performed.
In addition to the DNA template mixture and the oligonucleotide mixtures described above, the naica® multiplex PCR mix from Stilla Technology (Stilla Technologies, 1, Mail du Professeur Georges Mathé - 94800 Villejuif, France) was used.
The detailed mix preparation for each of the four experimental conditions was as follows:

| Reagent | Stock solution concentratio n | Target mix 100cp/$\mu$L/Linear reporter 0.25$\mu$M | | Target mix 100cp/$\mu$L/Linear reporter 0.1$\mu$M | |
|---|---|---|---|---|---|
| | | Final concentratio n | Volume ($\mu$L) | Final concentratio n | Volume ($\mu$L) |
| Water | | | 25,0 | | 28,5 |
| naica multiplex PCR MIX, buffer A | 10 | 1 | 5,5 | 1 | 5,5 |
| naica multiplex PCR MIX, buffer B | 100 | 4 | 2,2 | 4 | 2,2 |
| Primer mix | 5 | 0,5 | 5,5 | 0,50 | 5,5 |
| Mediator Probe mix | 5 | 0,50 | 5,5 | 0,50 | 5,5 |
| A strand mix | 5 | 0,25 | 2,8 | 0,10 | 1,1 |
| B strand mix | 5 | 0,28 | 3,0 | 0,11 | 1,2 |
| Target mix | 1000 | 100 | 5,5 | 100 | 5,5 |
| Total | | | 55,0 | | 55,0 |

| Reagent | Stock solution concentratio n | No Template Control/Linear reporter 0.25$\mu$M | | No Template Control/Linear reporter 0.1$\mu$M | |
|---|---|---|---|---|---|
| | | Final concentratio n | Volume ($\mu$L) | Final concentratio n | Volume ($\mu$L) |
| Water | | | 30,5 | | 34,0 |
| naica multiplex PCR MIX, buffer A | 10 | 1 | 5,5 | 1 | 5,5 |
| naica multiplex PCR MIX, buffer B | 100 | 4 | 2,2 | 4 | 2,2 |

(continued)

| Reagent | Stock solution concentratio n | No Template Control/Linear reporter 0.25µM | | No Template Control/Linear reporter 0.1µM | |
|---|---|---|---|---|---|
| | | Final concentratio n | Volume (µL) | Final concentratio n | Volume (µL) |
| Primer mix | 5 | 0,50 | 5,5 | 0,50 | 5,5 |
| Mediator Probe mix | 5 | 0,50 | 5,5 | 0,50 | 5,5 |
| A strand mix | 5 | 0,25 | 2,8 | 0,10 | 1,1 |
| B strand mix | 5 | 0,28 | 3,0 | 0,11 | 1,2 |
| Target mix | 1000 | 0 | 0 | 0 | 0 |
| Total | | | 55,0 | | 55,0 |

*Chip loading and dPCR run*

The PCR samples were loaded in Sapphire chips (Stilla Technologies, 1, Mail du Professeur Georges Mathé - 94800 Villejuif, France) and processed in the Geode instrument according to the standard procedure. The usual Sapphire partitioning and release programs were used with the cycling PCR program described below:

- Initial denaturation: 3 min at 95°C

- 60 cycles: 15 sec at 95°C then 30 sec. at 58°C

After the dPCR run, the chips were scanned using a Prism6 reader and the standard Sapphire scanning template (ScanningTemplate_Prism6_SapphireChip_naica-multiplex-PCR-MIX_Taqman_v1.4).

*Data analysis*

Data analysis was performed with the CrystalMiner software. To generate the compensation matrix, monocolor controls were used consisting in the mix of the universal reporters in combination with one single mediator probe to activate only one color.

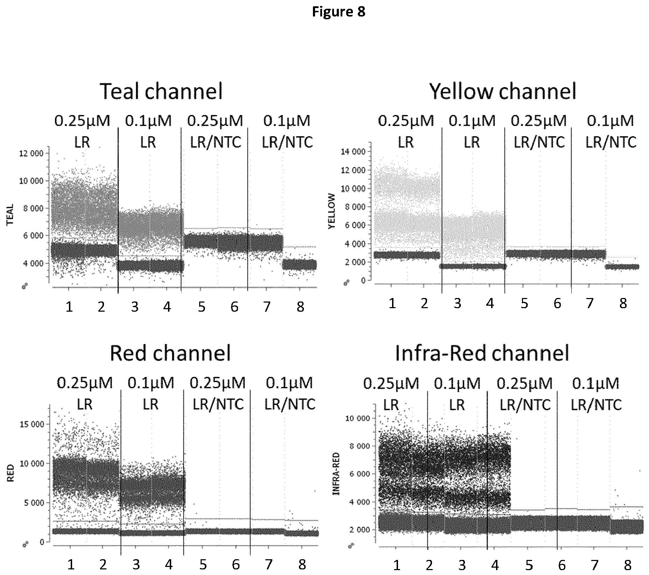

1D thresholds were set for each color channel to define negative and positive populations, as illustration in Figure 8.

Next, color assignment for each target sequence was set in the population editor section of the software. For example, PhiX174 was defined as the population positive in the teal and red channels and negative for all the other channels. After population edition, the results were exported and the quantification were reviewed. Experimentally measured concentrations were in good agreement with the theoretical concentration of 100 cp/µL for each of the six targets:

| Linear reporter concentration (µM) | phiX174 concentration (cp/µL) | Lambda concentration (cp/µL) | pUC18 concentration (cp/µL) | pBR322 concentration (cp/µL) | ALB concentration (cp/µL) | ESR1 concentration (cp/µL) |
|---|---|---|---|---|---|---|
| 0.25 | 91,80 | 98,40 | 88,65 | 122,50 | 142,85 | 83,15 |
| 0.1 | 94,90 | 104,40 | 92,50 | 130,75 | 107,95 | 88,55 |

EXAMPLE 3 : Use of three TaqMan® probes carrying a different color, for each Target Sequence, to effect the color combination method of the invention

*Materials and Methods*

Forward and reverse primers for the different Target Sequences (see table below) are used at a concentration of 0.5 μM.

Three probes with different fluorophores and quenchers are used for each target sequence at a concentration of 0.25 μM. The combination of colors applied per target sequence is the following: ESR1 L536P (Green, Yellow and Infrared), PhiX 174 (Yellow, Red and Infrared) and ALB (Yellow, Red and Teal).

| Name | Sequence | Dye | Quencher | Provider |
|------|----------|-----|----------|----------|
| PhiX174 F | TCTTTCCAAGCAACAGCAG (SEQ ID NO :1) | | | Eurogentec |
| PhiX174 R | AATACTGACCAGCCGTTTG A (SEQ ID NO : 2) | | | Eurogentec |
| PhiX174-ATTO700 | TCCGAGATTATGCGCCAAA TGC (SEQ ID NO :3) | ATTO700 | BHQ3 | Eurogentec |
| Phix174-ROX | TCCGAGATTATGCGCCAAA TGC (SEQ ID NO :73) | ROX | /Iowa Black RQ/ | IDT |
| Phix174-Cy5 | TCCGAGATTATGCGCCAAA TGC (SEQ ID NO :74) | Cy5 | /TAO//Io wa Black RQ/ | IDT |
| ESR1 530X F | CTGTACAGCATGAAGTGCA AG (SEQ ID NO :5) | | | Eurogentec |
| ESR1 530X R | GCATGTAGGCGGTGG (SEQ ID NO :6) | | | Eurogentec |
| ESR1 L536PATTO700 | TGCCCCCCTATGACCTGCT (SEQ ID NO :7) | ATTO700 | BHQ3 | IDT |
| ESR1 L536P-ROX | TGCCCCCCTATGACCTGCT (SEQ ID NO :8) | ROX | /Iowa Black RQ/ | IDT |
| ESR1 L536P-Cy3 | TGCCCCCCTATGACCTGCT (SEQ ID NO :75) | Cy3 | /Iowa Black RQ/ | IDT |
| ALB F | TGAAACATACGTTCCCAAA GAGTTT (SEQ ID NO :17) | | | Eurogentec |
| ALB R | CTCTCCTTCTCAGAAAGTG TGCATAT (SEQ ID NO :18) | | | Eurogentec |
| ALB P-YY | TGCTGAAACATTCACCTTC CATGCA (SEQ ID NO :19) | Yakima Yellow | /ZEN/ /Iowa Black FQ/ | IDT |

(continued)

| Name | Sequence | Dye | Quencher | Provider |
|---|---|---|---|---|
| ALB P-Cy5 | TGCTGAAACATTCACCTTC CATGCA (SEQ ID NO :20) | Cy5 | BHQ2 | Eurogentec |
| ALB P-ROX | TGCTGAAACATTCACCTTC CATGCA (SEQ ID NO :76) | ROX | /Iowa Black RQ/ | IDT |

Supplier details are as follows:

Kaneka Eurogentec SA - 5 Rue Bois Saint-Jean, 4102 Seraing, Belgium;
IDT - Integrated DNA Technologies, Inc. - 1710 Commercial Park - Coralville, Iowa 52241 - USA.

Target sequences for the assay were double-stranded synthetic DNAs ("gBlocks™" fragments from Integrated DNA Technologies, Inc. - 1710 Commercial Park - Coralville, Iowa 52241 - USA) corresponding to the amplicons, used at the following concentrations:

- ESR1 L536P: 450 cps/$\mu$L;.
- PhiX 174: 450 cps/$\mu$L;
- ALB: 600 cps/$\mu$L.

To perform the PCR, naica® multiplex PCR MIX (Stilla Technologies, 1, Mail du Professeur Georges Mathé - 94800 Villejuif, France) with fluorescein was added to the reaction. To increase the stability of the components, DMSO at 4% (vol/vol) was included in the reaction. 7 $\mu$l of the reaction were loaded in an Opal chip (Stilla Technologies, 1, Mail du Professeur Georges Mathé - 94800 Villejuif, France). Negative controls (with no DNA) and monocolor controls were added to control the reaction and to build the matrix compensation.

Partition and PCR cycles are performed in a Geode instrument (Stilla Technologies, 1, Mail du Professeur Georges Mathé - 94800 Villejuif, France).

The cycling PCR program has one initial step of denaturation at 95°C for 3 minutes followed by 60 cycles of 95°C for 15 seconds and 62°C for 30 seconds as denaturation and hybridization-elongation temperatures, respectively.

The chip is then released from the pressure (returned to atmospheric pressure) at 25°C and at 5 mbar/s. The chip is imaged using Prism6 imager (Stilla Technologies, 1, Mail du Professeur Georges Mathé - 94800 Villejuif, France) using the following exposure times:

- for blue 125 ms;
- for teal 400 ms;
- for green 125 ms;
- for yellow 175 ms;
- for red 500 ms; and
- for infrared 500 ms.

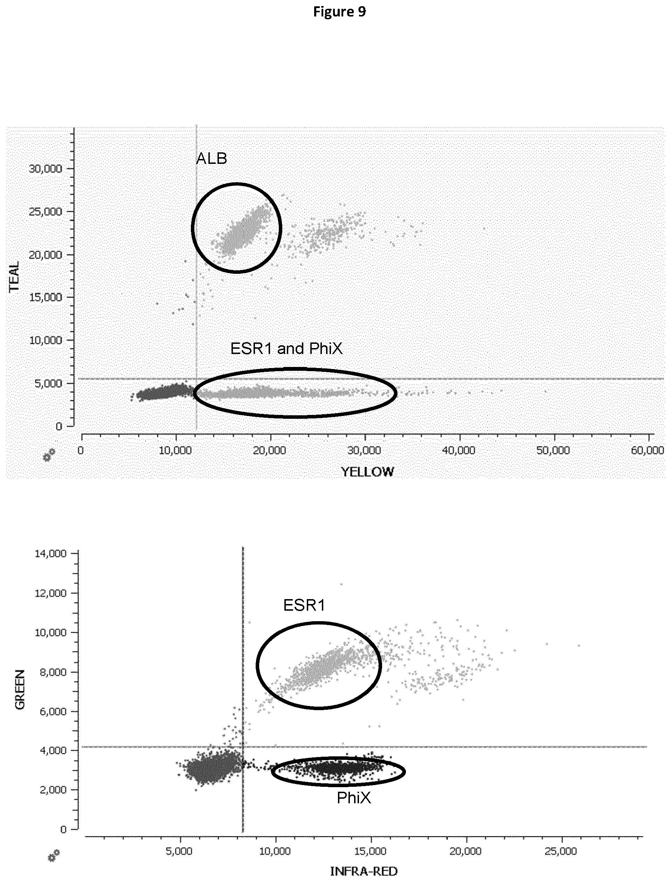

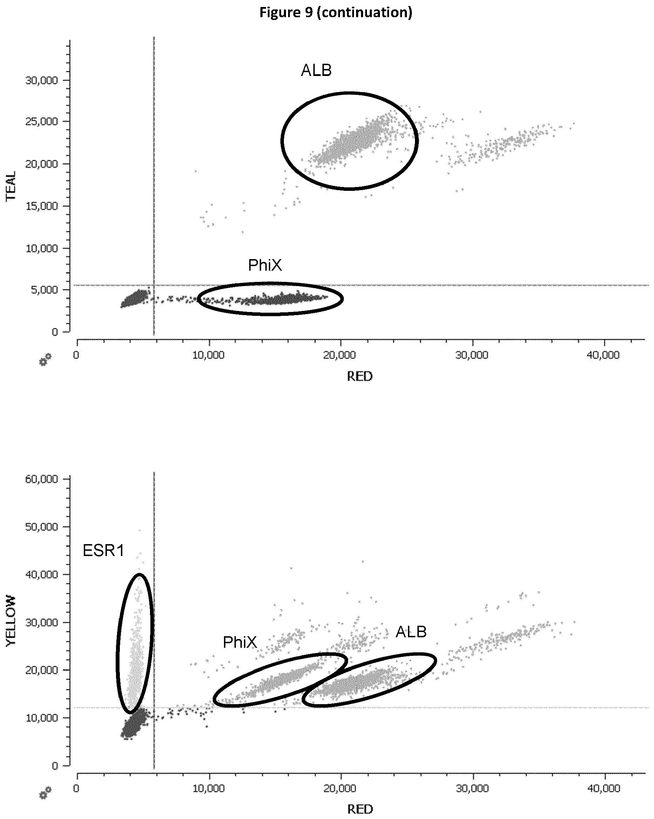

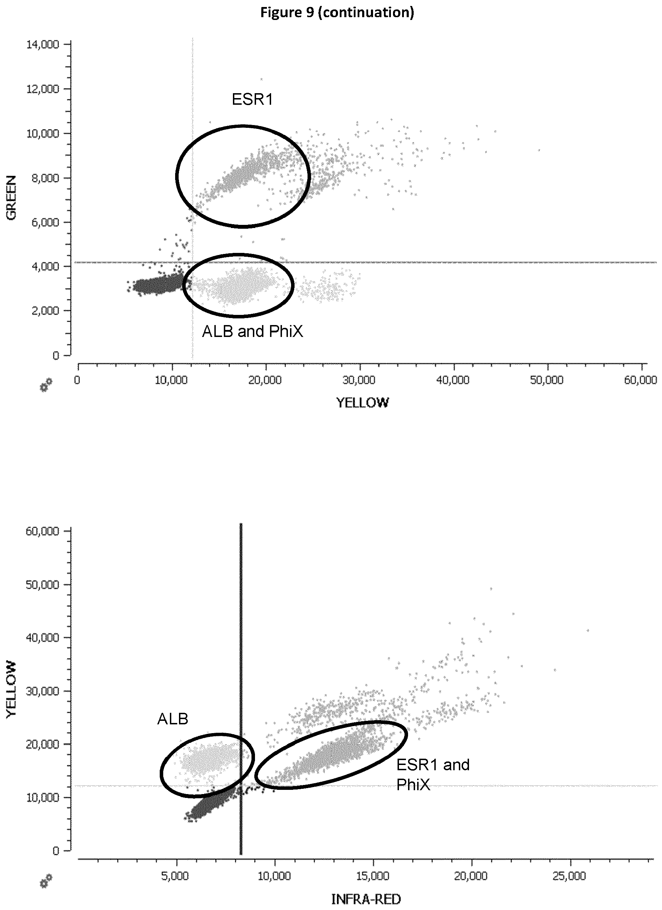

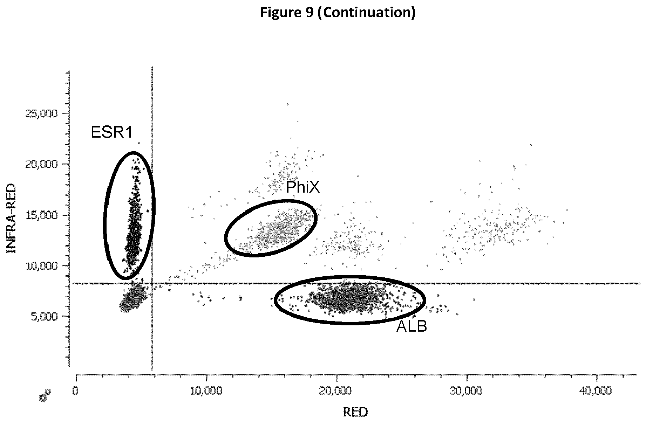

Data is analyzed using Crystal Miner software (Stilla Technologies, 1, Mail du Professeur Georges Mathé - 94800 Villejuif, France). For matrix compensation, monocolor controls are used. Thresholds are set to isolate negative droplets from the rest. This is typically done on 1D dot plot views. In the next step, the population editor in the Crystal Miner software is used to define the populations for each target sequence. For example, for the Phix174 target sequence, positive droplets are designated as the ones positive for infrared, red and yellow while the droplets displaying no fluorescence are counted as the negative droplets. Droplets displaying any other color combinations are excluded from the analysis (figure 9).

*Result*

By retrieving the number of positive droplets for each target sequence according to the method of the invention, it is possible to estimate the concentration of said target sequence in the sample, among other characteristics.

After population edition, the results were exported and the quantification were reviewed. Experimentally measured concentrations were in good agreement with the theoretical concentrations for each of the three targets:

|  | Concentration, cps/µL | Uncertainty percentage |
|---|---|---|
| ESR1 | 445.8 | 5.91 |
| Phyx | 459.3 | 5.82 |
| ALB | 606.1 | 5.02 |

EXAMPLE 4 : Use of 2 TaqMan® probes per target for effecting the color combination in conjunction with a single TaqMan® probe for detecting a last target: 10 target sequences - 2 colors per target sequence - and one gene on a single color

*Materials and Methods*

Forward and reverse primers for the different Target Sequences (see table below) are used at a concentration of 0.5 µM.

Two probes with different fluorophores and quenchers are used for each target sequence except for one target sequence. The details of the primers, probes with dyes and with quenchers, and the concentrations used, are listed in the table below.

The combination of colors applied per target sequence is the following: ESR1 L536P (Blue and Teal), PhiX 174 (Infrared and Yellow), PBR322 (Blue and Infrared), ALB (Blue and Green), Lambda (Yellow and Teal), puc18 (Infrared and Teal), TP53 R282W (Teal and Green), TP53 R248WT (Yellow and Green), MRM1 (Infrared and Green), ESR1 E380WT (Blue and Yellow) and De119ref (Green).

| Name | Sequence | Dye | Quencher | Provider | Concent ration (µM) |
|---|---|---|---|---|---|
| PhiX174 F | TCTTTCCAAGCAACAGCAG (SEQ ID NO :1) |  |  | Eurogentec | 0.5 |
| PhiX174 R | AATACTGACCAGCCGTTTGA (SEQ ID NO :2) |  |  | Eurogentec | 0.5 |
| PhiX174-ATTO700 | TCCGAGATTATGCGCCAAATGC (SEQ ID NO :3) | ATTO700 | BHQ3 | Eurogentec | 0.25 |
| Phix174-ROX | TCCGAGATTATGCGCCAAATGC (SEQ ID NO :73) | Yakima Yellow | /Iowa Black RQ/ | IDT | 0.25 |
| ESR1 530X F | CTGTACAGCATGAAGTGCAAG (SEQ ID NO :5) |  |  | Eurogentec | 0.5 |
| ESR1 530X R | GCATGTAGGCGGTGG (SEQ ID NO : 6) |  |  | Eurogentec | 0.5 |
| ESR1 L536P-FAM | TGCCCCCCTATGACCTGCT (SEQ ID NO :77) | FAM | /ZEN/ /Iowa Black FQ/ | IDT | 0.5 |
| ESR1 L536P-YY | TGCCCCCCTATGACCTGCT (SEQ ID NO :78) | YY | /ZEN/ /Iowa Black FQ/ | IDT | 0.25 |
| Lambda F | GCCATTGTTTCTCTGTGGAG (SEQ ID NO :9) |  |  | Eurogentec | 0.5 |
| Lambda R | TCACGGTTCAGTTGTTCACC (SEQ ID NO :10) |  |  | Eurogentec | 0.5 |

(continued)

| Name | Sequence | Dye | Quencher | Provider | Concentration (μM) |
|---|---|---|---|---|---|
| LambdaP-YY | TGATTGCCCGTCTCCGCT (SEQ ID NO :11) | Yakima Yellow | /ZEN/ /Iowa Black FQ/ | IDT | 0.25 |
| LambdaP-ROX | ACTGATTGCCCGTCTCCGCT (SEQ ID NO :12) | ROX | /Iowa Black RQ/ | IDT | 0.4 |
| pBR322 F | CGTTGATGCAATTTCTATGCG (SEQ ID NO :13) | | | Eurogentec | 0.5 |
| PBR322 R | TCGATAGTGGCTCCAAGTAG (SEQ ID NO :14) | | | Eurogentec | 0.5 |
| PBR322P-ATTO700 | CGCCCAGTCCTGCTCGCTTCG (SEQ ID NO :15) | ATTO700 | BHQ3 | Eurogentec | 0.25 |
| PBR322P-FAM | CGCCCAGTCCTGCTCGCTTCG (SEQ ID NO :79) | FAM | /ZEN/ /Iowa Black FQ/ | IDT | 0.4 |
| ALB F | TGAAACATACGTTCCCAAAGAG TTT (SEQ ID NO :17) | | | Eurogentec | 0.5 |
| ALB R | CTCTCCTTCTCAGAAAGTGTGC ATAT (SEQ ID NO :18) | | | Eurogentec | 0.5 |
| ALB P-FAM | TGCTGAAACATTCACCTTCCATG CA (SEQ ID NO :80) | FAM | BHQ1 | IDT | 0.4 |
| ALB P-Cy3 | TGCTGAAACATTCACCTTCCATG CA (SEQ ID NO :81) | Cy3 | /Iowa Black RQ/ | IDT | 0.25 |
| pUC18 L1 F | CCTGCAGGTCGACTCTAG (SEQ ID NO :21) | | | Eurogentec | 0.5 |
| pUC18 L1 R | TGAGCGGATAACAATTTCACA (SEQ ID NO :22) | | | Eurogentec | 0.5 |
| pUC18 P-ATTO700 | CCCGGGTACCGAGCTCGAATT (SEQ ID NO :82) | ATTO700 | BHQ3 | Eurogentec | 0.25 |
| pUC18 P-YY | CCCGGGTACCGAGCTCGAATT (SEQ ID NO :83) | YY | /ZEN/ /Iowa Black FQ/ | IDT | 0.25 |
| TP53 c. 830-840 Fw | TCCTATCCTGAGTAGTGGTAAT C (SEQ ID NO :84) | | | Eurogentec | 0.5 |
| TP53 c. 830-840 Rv | CCTTTCTTGCGGAGATTCTC (SEQ ID NO :85) | | | Eurogentec | 0.5 |
| Mut-spe2 TP53 R282W-YY | TGCGCCAGTCTCTCCCAG (SEQ ID NO :86) | YY | /ZEN/ /Iowa Black FQ/ | IDT | 0.25 |

(continued)

| Name | Sequence | Dye | Quencher | Provider | Concentration (μM) |
|------|----------|-----|----------|----------|---------------------|
| Mut-spe2 TP53 R282W-Cy3 | TGCGCCAGTCTCTCCCAG (SEQ ID NO :87) | Cy3 | /IowaBlack RQ-Sp/ | IDT | 0.5 |
| TP53 R248 Fw | CACCATCCACTACAACTACATG (SEQ ID NO :88) | | | Eurogentec | 0.5 |
| TP53 R248 Rv | TCCTGACCTGGAGTCTTC (SEQ ID NO :89) | | | Eurogentec | 0.5 |
| TP53 WT3 R248-ROX | CATGAACCGGAGGCCCATC (SEQ ID NO :90) | ROX | /IowaBlack RQ/ | IDT | 0.5 |
| TP53 WT3 R248-Cy3 | CATGAACCGGAGGCCCATC (SEQ ID NO :91) | Cy3 | /IowaBlack RQ-Sp/ | IDT | 0.25 |
| MRM1 F | GTGGATAAGGTCATCACCA (SEQ ID NO :92) | | | Eurogentec | 0.5 |
| MRM1 R | CAAGGTGCTTAGGAACTCG (SEQ ID NO :93) | | | Eurogentec | 0.5 |
| MRM1_P2-ATTO700 | ACGTCCCTCATTCTCTATGTGCC (SEQ ID NO :94) | ATTO700 | BHQ3 | Eurogentec | 0.25 |
| MRM1_P2-Cy3 | ACGTCCCTCATTCTCTATGTGCC (SEQ ID NO :95) | Cy3 | /IowaBlack RQ-Sp/ | IDT | 0.25 |
| ESR1 E380Q F | TGGATTTGACCCTCCATGAT (SEQ ID NO :96) | | | Eurogentec | 0.5 |
| ESR1 E380Q R | CCAGACGAGACCAATCATCA (SEQ ID NO :97) | | | Eurogentec | 0.5 |
| ESR1 E380_WT-FAM | TT+CTA+G+AATGTGCCTGG (SEQ ID NO :98) | FAM | BHQ1 | Eurogentec | 0.25 |
| ESR1 E380_WT-ROX | TT+CTA+G+AATGTGCCTGG (SEQ ID NO :98) | ROX | BHQ2 | Eurogentec | 0.25 |
| DEL19-Fw | GTG AGA AAG TTA AAA TTC CCG TC (SEQ ID NO :100) | | | Eurogentec | 0.5 |
| DEL19-Rv | CCA CAC AGC AAA GCA GAA AC (SEQ ID NO :101) | | | Eurogentec | 0.5 |
| DEL19-REF-Cy3 | CA CAT CGA GG ATT TCC TTG TTG GC (SEQ ID NO :102) | Cy3 | /IowaBlack RQ-Sp/ | IDT | 0.15 |

In SEQ ID NO:98, the "+" correspond to LNA nucleic acids in position 2, 5 and 6 of SEQ ID NO:99.

Supplier details are as follows:

- Kaneka Eurogentec SA - 5 Rue Bois Saint-Jean, 4102 Seraing, Belgium;
- IDT - Integrated DNA Technologies, Inc. - 1710 Commercial Park - Coralville, Iowa 52241 - USA.

Target sequences for the assay were double-stranded synthetic DNAs ("gBlocksTM" fragments from Integrated DNA Technologies, Inc. - 1710 Commercial Park - Coralville, Iowa 52241 - USA) corresponding to the amplicons, used at an approximate concentration of 100 cps/$\mu$L, except for ESR1 E380WT where the concentration was ca. 200 cps/$\mu$L and for ALB where the concentration was ca. 160 cps/$\mu$L.

To perform the PCR, naica® PCR MIX without fluorescein (Stilla Technologies, 1, Mail du Professeur Georges Mathé - 94800 Villejuif, France) was added to the reaction. To increase the stability of the components, DMSO at 4% (vol/vol) was included in the reaction. 7 $\mu$L of the reaction were loaded in an Opal chip (Stilla Technologies, 1, Mail du Professeur Georges Mathé - 94800 Villejuif, France). Negative controls (with no DNA) and monocolor controls were added to control the reaction and to build the matrix compensation.

Partition and PCR cycles are performed in a Geode instrument (Stilla Technologies, 1, Mail du Professeur Georges Mathé - 94800 Villejuif, France).

The cycling PCR program has one initial step of denaturation at 95°C for 3 minutes followed by 60 cycles of 95°C for 15 seconds and 62°C for 30 seconds as denaturation and hybridization-elongation temperatures, respectively.

The chip is then released from the pressure (returned to atmospheric pressure) at 25°C and at 5 mbar/s. The chip is imaged using Prism6 imager (Stilla Technologies, 1, Mail du Professeur Georges Mathé - 94800 Villejuif, France) using the following exposure times:

- for blue 125 ms;

- for teal 350 ms;

- for green 125 ms;

- for yellow 150 ms;

- for red 500 ms; and

- for infrared 500 ms.

Data is analyzed using the Crystal Miner software (Stilla Technologies, 1, Mail du Professeur Georges Mathé - 94800 Villejuif, France). For matrix compensation, monocolor controls are used.

Thresholds are set to isolate negative droplets from the rest. This is typically done on 1D dot plot views.

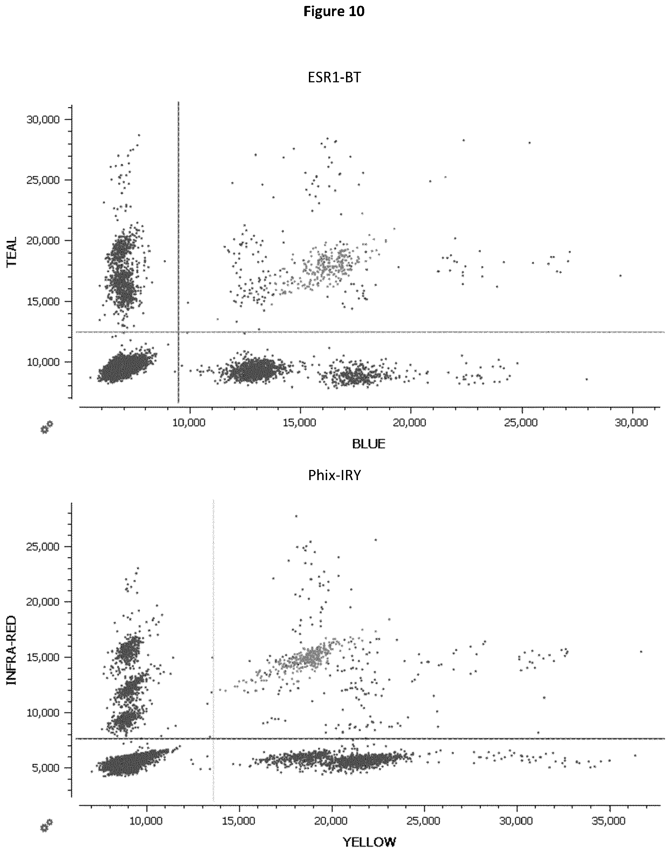

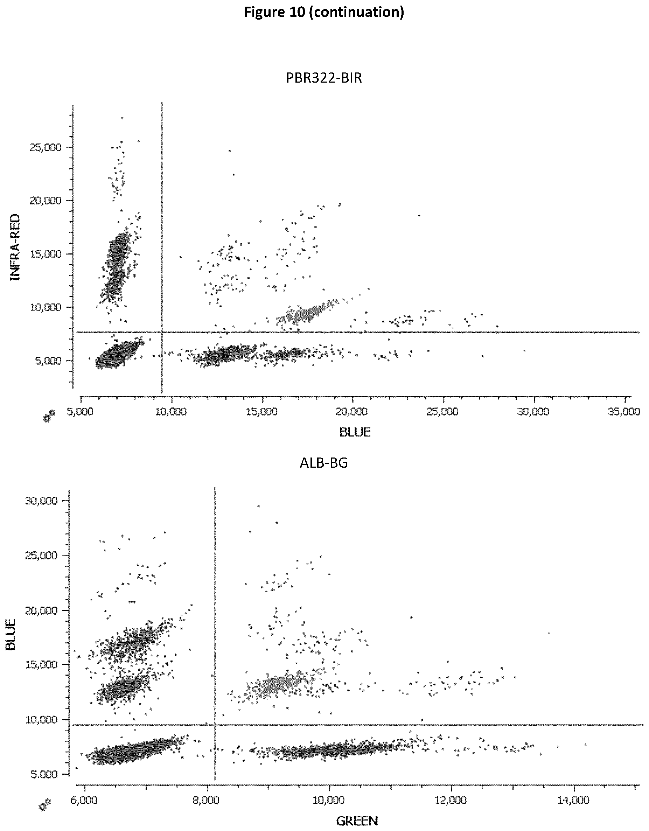

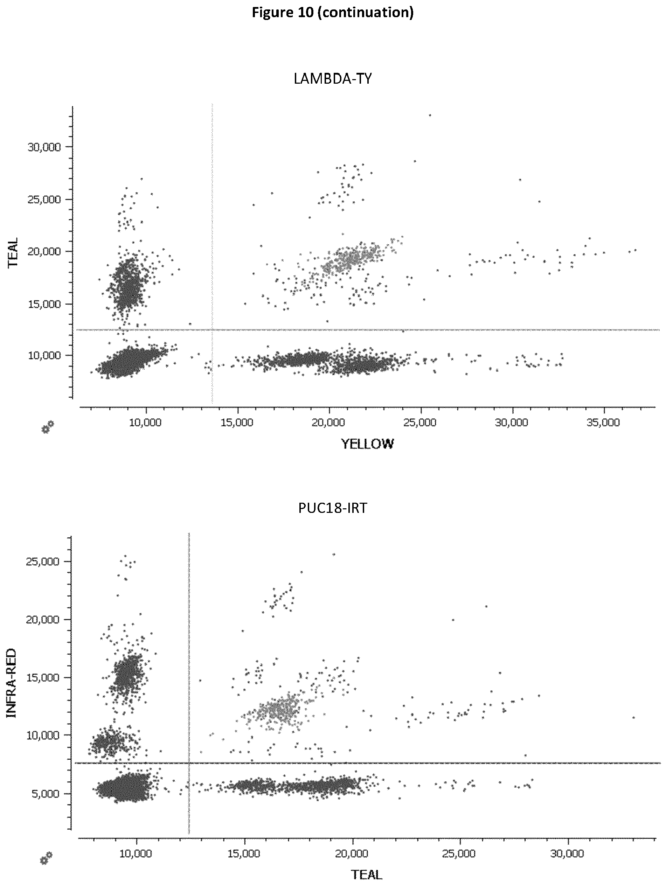

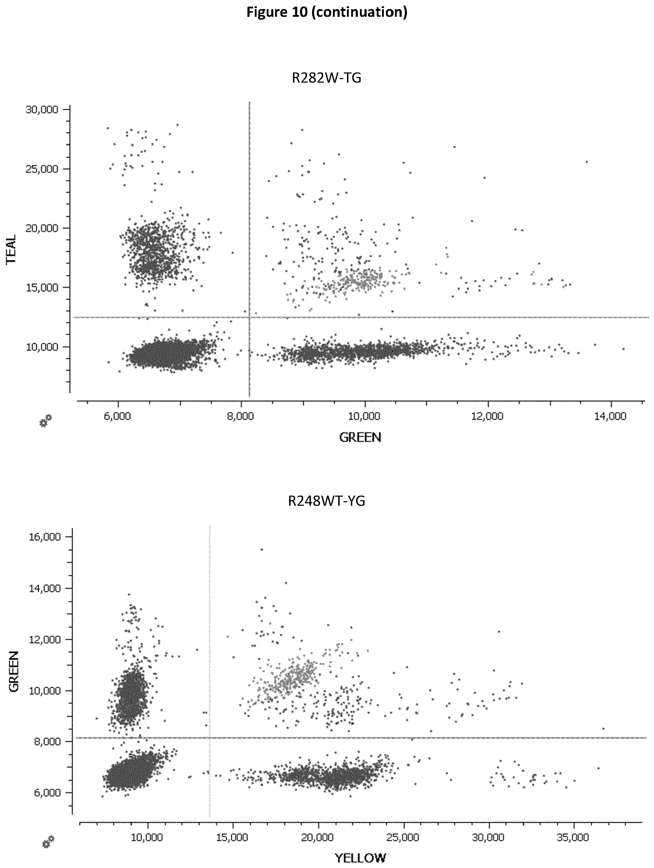

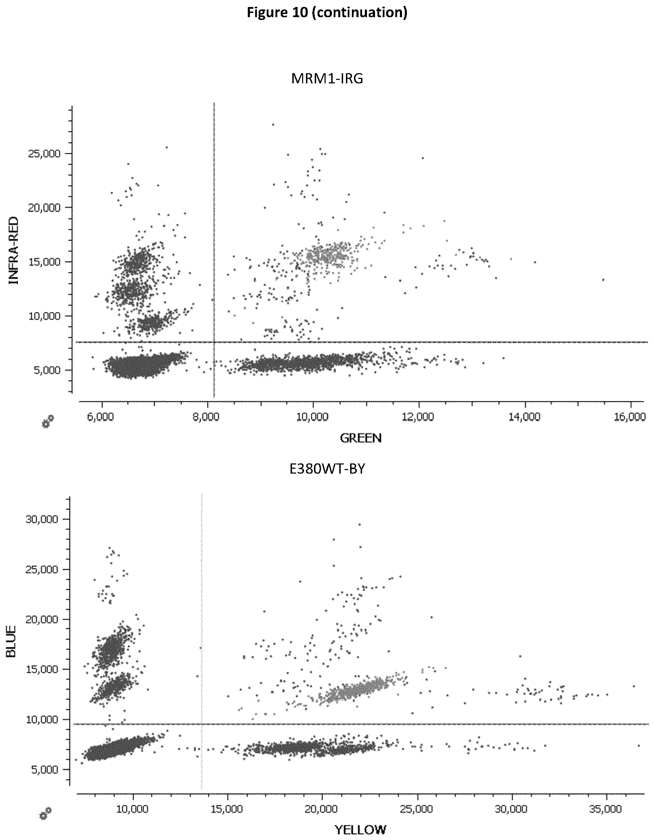

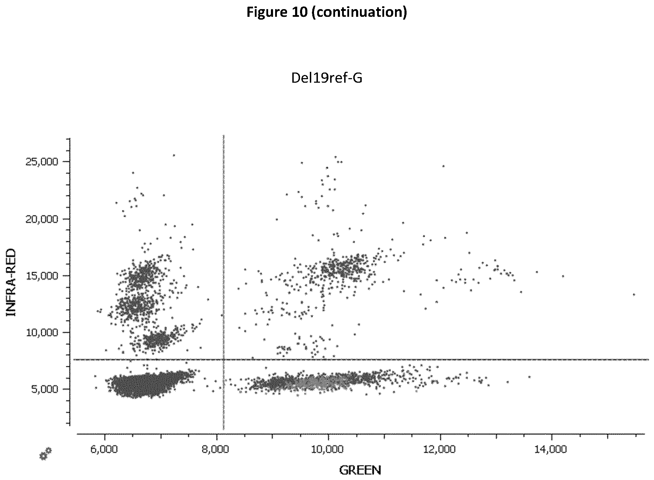

In the next step, the population editor in the Crystal Miner software is used to define the populations for each target sequence. For example, for the Phix174 target sequence, positive droplets are designated as the ones positive for infrared and yellow. In addition, the droplets displaying no fluorescence are counted as the negative droplets. Droplets displaying any other color combinations are excluded from the analysis (figure 10).

*Results*

In this way, it is possible to retrieve the number of positive droplets for the target sequence and to estimate the concentration of said target sequence in the sample, among other characteristics.

BIBLIOGRAPHIC REFERENCES

[1] Q. Zhong, S. Bhattacharya, S. Kotsopoulos, J. Olson, V. Taly, A.D. Griffiths, D.R. Link, J.W. Larson, Multiplex digital PCR: breaking the one target per color barrier of quantitative PCR, Lab Chip 11 (13) (2011) 2167;

[2] V. Taly, D. Pekin, L. Benhaim, S.K. Kotsopoulos, D. Le Corre, X. Li, I. Atochin, D.R. Link, A.D. Griffiths, K. Pallier, H. Blons, O. Bouche, B. Landi, J.B. Hutchison, P. Laurent-Puig, Multiplex Picodroplet Digital PCR to Detect KRAS Mutations in Circulating DNA from the Plasm a of Colorectal Cancer Patients, Clin. Chem. 59 (12) (2013) 1722-1731

[3] A.S. Whale, J.F. Huggett, S. Tzonev, Fundamentals of multiplexing with digital PCR, BDQ (May) (2016)

[4] Loic Lindner, Pauline Cayrou, Sylvie Jacquot, Marie-Christine Birling, Yann Herault, Guillaume Pavlovic, Reliable and robust droplet digital PCR (ddPCR) and RT-ddPCR protocols for mouse studies, Methods, Volume 191, 2021, Pages 95-106,

[5] T. Weissensteiner, J.S. Lanchbury, Strategy for controlling preferential amplification and avoiding false negatives in PCR typing, BioTechniques 21 (December (6)) (1996) 1102-1108

[6] Marras SAE, Tyagi S, Antson DO, Kramer FR. Color-coded molecular beacons for multiplex PCR screening assays. PLoS One. 2019 Mar 18;14(3):e0213906

[7] Alexandra S. Whale, Jim F. Huggett, Svilen Tzonev, Fundamentals of multiplexing with digital PCR, Biomolecular Detection and Quantification, Volume 10, 2016, Pages 15-23

[8] Benjamin J Hindson 1, Kevin D Ness, Donald A Masquelier, Phillip Belgrader, Nicholas J Heredia, Anthony J Makarewicz, Isaac J Bright, Michael Y Lucero, Amy L Hiddessen, Tina C Legler, Tyler K Kitano, Michael R Hodel, Jonathan F Petersen, Paul W Wyatt, Erin R Steenblock, Pallavi H Shah, Luc J Bousse, Camille B Troup, Jeffrey C Mellen, Dean K Wittmann, Nicholas G Erndt, Thomas H Cauley, Ryan T Koehler, Austin P So, Simant Dube, Klint A Rose, Luz Montesclaros, Shenglong Wang, David P Stumbo, Shawn P Hodges, Steven Romine, Fred P Milanovich, Helen E White, John F Regan, George A Karlin-Neumann, Christopher M Hindson, Serge Saxonov, Bill W Colston, High-throughput droplet digital PCR system for absolute quantitation of DNA copy number, Anal Chem. 2011 Nov 15;83(22):8604-10. doi: 10.1021/ac202028g. Epub 2011 Oct 28.

[9] J Madic, A Zocevic, V Senlis, E Fradet, B Andre, S Muller, R Dangla, M E Droniou, Three-color crystal digital PCR, Biomol Detect Quantif. 2016 Nov 3;10:34-46. doi: 10.1016/j.bdq.2016.10.002

[10] Hinson et al., 2011, Anal. Chem. 83:8604-8610; Pinheiro et al., 2012, Anal. Chem. 84: 1003-1011

[11] Qiuying Huang, Dongmei Chen, Chen Du, Qiaoqiao Liu, Su Lin, Lanlan Liang, Ye Xu, Yiqun Liao, Qingge Li, Highly multiplex PCR assays by coupling the 5'-flap endonuclease activity of Taq DNA polymerase and molecular beacon reporters, Proc Natl Acad Sci USA. 2022 Mar 1;119(9):e2110672119.

[12] James A. Richardson, Trevor Morgan, Michael Andreou and Tom Brown, Use of a large Stokes-shift fluorophore to increase the multiplexing capacity of a point-of-care DNA diagnostic device, Analyst, issue 13, 2013

[13] Maksim V Sednev, Vladimir N Belov and Stefan W Hell, Fluorescent dyes with large Stokes shifts for super-resolution optical microscopy of biological objects: a review, Methods Appl. Fluoresc. 3 042004

[14] Basu AS; Digital Assays Part I: Partitioning Statistics and Digital PCR; SLAS Technol., August 2017; 22(4); pages: 369-386

## Claims

1. A multiplexed digital PCR (dPCR) method to detect and/or quantify, in a biological sample comprising nucleic acid molecules, the presence of a set of at least five different nucleic acid target sequences (TS), said method comprising:

   a) contacting the sample with fluorophore-carrying probes capable of directly or indirectly binding to the TS; wherein each Target Sequence $i$ (TS$i$) is **characterized by** $k_i$ different fluorophore types, $k_i$ being superior or equal to 2;
   b) separating the sample into a set of partitions;
   c) exponentially amplifying the TS in the presence of a PCR reaction mix;
   d) measuring, for each partition, the intensity of fluorescence for each fluorophore type;
   e) counting, for each fluorophore type,

   - the partitions having a fluorescence signal below a positivity threshold for the said fluorophore type,
   - the partitions having a fluorescence signal above said positivity threshold for the said fluorophore type,

   So as to determine:

   • $N_0$ = the total number of partitions having a fluorescence signal below said positivity threshold for all the fluorophore types,
   • $N_i$ = the total number of partitions having a fluorescence signal above the positivity threshold only for the

$k_i$ fluorophore types characterizing TS$i$.

f) processing the data collected in step e) in order to quantify the concentration of the at least five TS$i$ in said biological sample.

2. The method of claim 1, wherein, for each target sequence TS$i$, the calculation of its concentration $Ci$ in step f) is performed according to Poisson's law, preferably using the equation $$C_i = -\frac{d}{v}\ln(1 - \frac{N_i}{N_0 + N_i})$$ where v is the volume of the partition, and d is the dilution factor used to dilute the biological sample to the microfluidic well where dPCR is performed.

3. The method of claim 1 or 2, wherein step e) further contains the determination of $N_1$ corresponding to the total number of partitions having a fluorescence signal above the positivity threshold for only one fluorophore type, and wherein $Ci$ is calculated in step f) by using the equation: $$C_i = -\frac{d}{v}\ln(1 - \frac{N_i}{N_0 + N_1 + N_i})$$

4. The method of any one of claims 1-3, wherein step d) further comprises setting the positivity threshold intensity for each fluorophore type, said positivity threshold being preferably defined on 1D plot views, for each fluorophore type separately, and corresponds to the intensity value distinguishing partitions displaying a high intensity of one fluorophore type from those displaying a low intensity of the same fluorophore type.

5. The method of any one of claims 1-4, wherein $k_i$ is constant for all Target Sequences $i$, and the partitions having a fluorescence signal above the positivity threshold for $k_i+1$ fluorophore types are not taken into account.

6. The method of any one of claims 1-5, wherein step d) uses, for measuring the intensity of the fluorophore types, a number $n$ of different fluorescence detection channels which is superior or equal to 4, preferably superior or equal to 5, more preferably is 6 or 7.

7. The method of any one of claims 1-6, wherein, if $M$ is the total number of different fluorophore types carried by all the probes, and if $n$ is the number of different fluorescence detection channels used in step d) for measuring the intensity of the fluorophore types, then $M$ is inferior or equal to $n$.

8. The method of any one of claims 1-7, wherein each of said at least five TS is **characterized by** a unique combination of exactly two different fluorophore types.

9. The method of any one of claims 1-8, wherein said fluorophore-carrying probes are probes that bind directly to the TS$i$, said probes being preferably TaqMan® probes, each TS$i$ being specifically recognized by $k_i$ probes each carrying a different fluorophore type, the combination of which characterizing each TS$i$ in a unique manner.

10. The method of any one of claims 1-8, wherein said fluorophore-carrying probes bind to mediator probes that directly bind to the TS, and, once bound, can release a tag that is complementary to a tag complementary sequence (TCS) located on a fluorescent detection molecule carrying a quencher group and a fluorophore, whose fluorescence is modified as a result of the hybridization or of the elongation of the tag sequence of the mediator probes, once released, on the TCS on said detection molecule.

11. The method of claim 10, wherein said fluorescent detection molecule is a universal reporter detection molecule, preferably a molecular beacon reporter or a complementary single-stranded fluorescent detection reporter (CSSFR).

12. The method of any one of claims 1-11, wherein, for each partition, the fluorescence signal intensity from the fluorophore types is measured using the signals from the fluorescence detection channels used in step d) for measuring the intensity of the fluorophore types, by applying a fluorescence compensation matrix that has been previously generated.

13. The method of any one of claims 1-12, wherein the concentration of the sample before partitioning is adjusted so that the maximum concentration of the TS is of 200 cp/$\mu$L.

**14.** The method of any one of claims 1-13, wherein at least one of the fluorophore type is one of the Large Stokes Shift Dyes (LSSD).

**15.** Computer program product comprising instructions which, when the program is executed by a computer, cause the computer to carry out the steps e) and f) of the method according any one of claims 1-14.

Figure 1

## Figure 1 (continuation)

## Figure 2

### A

Dilution series in ALB without any other target sequence

y = 1,0021x
R² = 0,9988

### B

Dilution series in ALB with 5 target sequences at 100 cps/µl

y = 1,0305x
R² = 0,9999

**Figure 2 (Continuation)**

**C**

Internal Control target sequence with increasing background gene

Figure 3

Assay sensivity for Target i vs concentration of other targets

Figure 4

Figure 5

Elongation of the primer and
release of the flap sequence

MB reporter

Elongation of the flap sequence
and MB unfolding

5′

3′

Mediator probe

Flap        Target sequence
sequence       specific

Polymerase
cleavage site

Polymerase

**Figure 6**

# Figure 6 (continuation)

Figure 7

Figure 8

**Figure 9**

**Figure 9 (continuation)**

**Figure 9 (continuation)**

**Figure 9 (Continuation)**

**Figure 10**

ESR1-BT

Phix-IRY

**Figure 10 (continuation)**

PBR322-BIR

ALB-BG

**Figure 10 (continuation)**

LAMBDA-TY

PUC18-IRT

**Figure 10 (continuation)**

R282W-TG

R248WT-YG

**Figure 10 (continuation)**

MRM1-IRG

E380WT-BY

**Figure 10 (continuation)**

Del19ref-G

Europäisches
Patentamt
European
Patent Office
Office européen
des brevets

# EUROPEAN SEARCH REPORT

**Application Number**

EP 22 30 6683

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/189486 A1 (ANDRÉ BARBARA [FR] ET AL) 24 June 2021 (2021-06-24) | 1-9, 13-15 | INV. C12Q1/6813 C12Q1/6851 |
| Y | * [0007]-[0012], [0022], [0023], [0084], Fig. 4A-4B * | 10-12 | |
| A,D | BENJAMIN J. HINDSON ET AL: "High-Throughput Droplet Digital PCR System for Absolute Quantitation of DNA Copy Number", ANALYTICAL CHEMISTRY, vol. 83, no. 22, 15 November 2011 (2011-11-15), pages 8604-8610, XP055531826, US ISSN: 0003-2700, DOI: 10.1021/ac202028g * abstract, Fig. 1 * | 1-15 | |
| Y | US 2020/140931 A1 (LI QINGGE [CN] ET AL) 7 May 2020 (2020-05-07) * claim 1, Fig. 1A and [0031] * | 10,11 | |
| Y | FALTIN B ET AL: "Mediator probe PCR: a novel approach for detection of real-time PCR based on lavbel-free primary probes and standardized secondary universal fluorogenic reporters", CLINICAL CHEMISTRY, OXFORD UNIVERSITY PRESS, US, vol. 58, 1 November 2012 (2012-11-01), pages 1546-1556, XP002694250, ISSN: 0009-9147, DOI: 10.1373/CLINCHEM.2012.186734 [retrieved on 2012-08-24] * abstract, Fig. 1 * | 10,11 | TECHNICAL FIELDS SEARCHED (IPC)<br><br>C12Q |
| Y | US 2015/308958 A1 (LEMIEUX HUGO [CA] ET AL) 29 October 2015 (2015-10-29) * [0008], Tab. 4 * | 12 | |

The present search report has been drawn up for all claims

| Place of search | Date of completion of the search | Examiner |
|---|---|---|
| Munich | 27 March 2023 | Lapopin, Laurence |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                                  
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 30 6683

This annex lists the patent family members relating to the patent documents cited in the above-mentioned European search report.
The members are as contained in the European Patent Office EDP file on
The European Patent Office is in no way liable for these particulars which are merely given for the purpose of information.

27-03-2023

| Patent document cited in search report | | | Publication date | Patent family member(s) | | | Publication date |
|---|---|---|---|---|---|---|---|
| US 2021189486 | A1 | | 24-06-2021 | CN | 115135776 | A | 30-09-2022 |
| | | | | EP | 4081661 | A1 | 02-11-2022 |
| | | | | US | 2021189486 | A1 | 24-06-2021 |
| | | | | US | 2023057726 | A1 | 23-02-2023 |
| | | | | WO | 2021130271 | A1 | 01-07-2021 |
| US 2020140931 | A1 | | 07-05-2020 | AU | 2018258752 | A1 | 21-02-2019 |
| | | | | CN | 108823287 | A | 16-11-2018 |
| | | | | EP | 3640340 | A1 | 22-04-2020 |
| | | | | JP | 6661835 | B2 | 11-03-2020 |
| | | | | JP | 2019528772 | A | 17-10-2019 |
| | | | | KR | 20190089944 | A | 31-07-2019 |
| | | | | US | 2020140931 | A1 | 07-05-2020 |
| | | | | US | 2022017949 | A1 | 20-01-2022 |
| | | | | WO | 2018196842 | A1 | 01-11-2018 |
| US 2015308958 | A1 | | 29-10-2015 | BR | 112015013166 | A2 | 11-07-2017 |
| | | | | CA | 2862766 | A1 | 12-06-2014 |
| | | | | CN | 105008878 | A | 28-10-2015 |
| | | | | EP | 2929308 | A1 | 14-10-2015 |
| | | | | JP | 6501714 | B2 | 17-04-2019 |
| | | | | JP | 2016502670 | A | 28-01-2016 |
| | | | | US | 2015308958 | A1 | 29-10-2015 |
| | | | | WO | 2014087380 | A1 | 12-06-2014 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2776585 A

- WO 2022063845 A

**Non-patent literature cited in the description**

- **Q. ZHONG ; S. BHATTACHARYA ; S. KOTSOPOULOS ; J. OLSON ; V. TALY ; A.D. GRIFFITHS ; D.R. LINK ; J.W. LARSON.** Multiplex digital PCR: breaking the one target per color barrier of quantitative PCR. *Lab Chip,* 2011, vol. 11 (13), 2167

- **V. TALY, D. PEKIN ; L. BENHAIM ; S.K. KOTSOPOULOS ; D. LE CORRE ; X. LI ; I. ATOCHIN ; D.R. LINK ; A.D. GRIFFITHS ; K. PALLIER ; H. BLONS.** Multiplex Picodroplet Digital PCR to Detect KRAS Mutations in Circulating DNA from the Plasm a of Colorectal Cancer Patients. *Clin. Chem.,* 2013, vol. 59 (12), 1722-1731

- **A.S. WHALE ; J.F. HUGGETT ; S. TZONEV.** *Fundamentals of multiplexing with digital PCR, BDQ,* May 2016

- **LOIC LINDNER ; PAULINE CAYROU ; SYLVIE JACQUOT ; MARIE-CHRISTINE BIRLING ; YANN HERAULT ; GUILLAUME PAVLOVIC.** Reliable and robust droplet digital PCR (ddPCR) and RT-ddPCR protocols for mouse studies. *Methods,* 2021, vol. 191, 95-106

- **T. WEISSENSTEINER ; J.S. LANCHBURY.** Strategy for controlling preferential amplification and avoiding false negatives in PCR typing. *BioTechniques,* December 1996, vol. 21 (6), 1102-1108

- **MARRAS SAE ; TYAGI S ; ANTSON DO ; KRAMER FR.** Color-coded molecular beacons for multiplex PCR screening assays. *PLoS One,* 18 March 2019, vol. 14 (3), e0213906

- **ALEXANDRA S. WHALE ; JIM F. HUGGETT ; SVILEN TZONEV.** Fundamentals of multiplexing with digital PCR. *Biomolecular Detection and Quantification,* 2016, vol. 10, 15-23

- **BENJAMIN J HINDSON 1 ; KEVIN D NESS ; DONALD A MASQUELIER ; PHILLIP BELGRADER ; NICHOLAS J HEREDIA ; ANTHONY J MAKAREWICZ ; ISAAC J BRIGHT ; MICHAEL Y LUCERO ; AMY L HIDDESSEN ; TINA C LEGLER.** High-throughput droplet digital PCR system for absolute quantitation of DNA copy number. *Anal Chem.,* 28 October 2011, vol. 83 (22), 8604-10

- **J MADIC ; A ZOCEVIC ; V SENLIS ; E FRADET ; B ANDRE ; S MULLER ; R DANGLA.** M E Droniou, Three-color crystal digital PCR. *Biomol Detect Quantif,* 03 November 2016, vol. 10, 34-46

- **HINSON et al.** *Anal. Chem.,* 2011, vol. 83, 8604-8610

- **PINHEIRO et al.** *Anal. Chem.,* 2012, vol. 84, 1003-1011

- **QIUYING HUANG ; DONGMEI CHEN ; CHEN DU ; QIAOQIAO LIU ; SU LIN ; LANLAN LIANG ; YE XU ; YIQUN LIAO ; QINGGE LI.** Highly multiplex PCR assays by coupling the 5'-flap endonuclease activity of Taq DNA polymerase and molecular beacon reporters. *Proc Natl Acad Sci USA.,* 01 March 2022, vol. 119 (9

- **JAMES A. RICHARDSON ; TREVOR MORGAN ; MICHAEL ANDREOU ; TOM BROWN.** Use of a large Stokes-shift fluorophore to increase the multiplexing capacity of a point-of-care DNA diagnostic device. *Analyst,* 2013, (13

- **MAKSIM V SEDNEV ; VLADIMIR N BELOV ; STEFAN W HELL.** Fluorescent dyes with large Stokes shifts for super-resolution optical microscopy of biological objects: a review. *Methods Appl. Fluoresc,* vol. 3, 042004

- **BASU AS.** Digital Assays Part I: Partitioning Statistics and Digital PCR. *SLAS Technol.,* August 2017, vol. 22 (4), 369-386